# EUROPEAN PATENT SPECIFICATION

(11) **EP 3 570 839 B1**
(45) Date of publication and mention of the grant of the patent: **02.12.2020**
(21) Application number: 18702877.4
(22) Date of filing: 18.01.2018
(51) Int. Cl.: A61K 31/4545, A61K 9/14, A61P 35/00

(54) **SOLID DISPERSIONS OF (R)-N-((4-METHOXY-6-METHYL-2-OXO-1,2-DIHYDROPYRIDIN-3-YL)METHYL)-2-METHYL-1-(1-(1-(2,2,2-TRIFLUOROETHYL)PIPERIDIN-4-YL)ETHYL)-1H-INDOLE-3-CARBOXAMIDE**
FESTE DISPERSIONEN VON (R)-N-((4-METHOXY-6-METHYL-2-OXO-1,2-DIHYDROPYRIDIN-3-YL)METHYL)-2-METHYL-1-(1-(1-(2,2,2-TRIFLUORETHYL)PIPERIDIN-4-YL)ETHYL)-1H-INDOL-3-CARBOXAMID
DISPERSIONS SOLIDES DE (R)-N-((4-MÉTHOXY-6-MÉTHYL-2-OXO-1,2-DIHYDROPYRIDIN-3-YL)MÉTHYL)-2-MÉTHYL-1-(1-(1-(2,2,2-TRIFLUOROÉTHYL)PIPÉRIDIN-4-YL)ÉTHYL)-1H-INDOLE-3-CARBOXAMIDE

(30) Priority: 20.01.2017 US 201762448486 P
(43) Date of publication of application: 27.11.2019
(73) Proprietor: Constellation Pharmaceuticals, Inc., Cambridge, MA 02142 (US)
(72) Inventor: ARRIGO, Alisha, Longmont CO 80503 (US); CORSON, Donald, San Francisco CA 94105 (US); HARMANGE, Jean-Christophe, Andover MA 01810 (US)
(74) Representative: Berger, Axel Bernhard
(86) International application number: PCT/US2018/014158
(87) International publication number: WO 2018/136596

(56) References cited:
- VASWANI ET AL.: "IDENTIFICATION OF (R)-N-((4-METHOXY-6-METHYL-2-OXO-1,2-DIHYD ROPYRIDIN-3-YL)METHYL)-2-METHYL-1-(1-(1-(2 ,2,2,-TRIFLUOROETHYL)PIPERIDIN-4-YL)ETHYL) -1H-INDOLE-3-CARBOXAMIDE(CPI-1205), A POTENT AND SELECTIVE INHIBITOR OF HISTONE METHYLTRANSFERASE EZH2, SUITABLE FOR PHASE I CLINICAL TRIALS", JOURNAL OF MEDICINAL CHEMISTRY, vol. 59, 2016, pages 9928-9941, XP002779342,
- VASCONCELOS T; SARMENTO B; COSTA P: "Solid dispersions as strategy to improve oral bioavailability of poor water soluble drugs", DRUG DISCOVERY TODAY, vol. 12, 2007, pages 1068-1075, XP22370275, cited in the application
- QIAN F. ET AL.: "DRUG-POLYMER SOLUBILITY AND MISCIBILITY: STABILITY CONSIDERATION AND PRACTICAL CHALLENCES IN AMORPHOUS SOLID DISPERSION DEVELOPMENT", JOURNAL OF PHARMACEUTICAL SCIENCES, vol. 99, no. 7, July 2010 (2010-07), pages 2941-2947, XP002779344,

## Description

### TECHNICAL FIELD OF THE INVENTION

Provided herein are solid dispersions of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide.

### BACKGROUND OF THE INVENTION

Histone methylation plays a critical role in the regulation of gene expression in eukaryotes. Methylation affects chromatin structure and has been linked to both activation and repression of transcription (Zhang and Reinberg, Genes Dev. 15:2343-2360, 2001). Enzymes that catalyze attachment and removal of methyl groups from histones are implicated in gene silencing, embryonic development, cell proliferation, and other processes.

One class of histone methylases is characterized by the presence of a Suppressor of Variegation Enhancer of Zeste Trithorax (SET) domain, comprising about 130 amino acids. Enhancer of Zeste Homolog 2 (EZH2) is an example of a human SET-domain containing methylase. EZH2 associates with EED (Embryonic Ectoderm Development) and SUZ12 (suppressor of zeste 12 homolog) to form a complex known as PRC2 (Polycomb GroupRepressive Complex 2) having the ability to tri-methylate histone H3 at lysine 27 (Cao and Zhang, Mol. Cell 15:57-67, 2004). PRC2 complexes can also include RBAP46 and RBAP48 subunits.

The oncogenic activities of EZH2 have been shown by a number of studies. In cell line experiments, over-expression of EZH2 induces cell invasion, growth in soft agar, and motility while knockdown of EZH2 inhibits cell proliferation and cell invasion (Kleer et al., 2003, Proc. Nat. Acad. Sci. USA 100:11606-11611; Varambally et al., (2002), "The polycomb group protein EZH2 is involved in progression of prostate cancer," Nature 419, 624-629). It has been shown that EZH2 represses the expression of several tumor suppressors, including E-cadherin, DAB2IP and RUNX3 among others. In xenograft models, EZH2 knockdown inhibits tumor growth and metastasis. Recently, it has been shown that down modulation of EZH2 in murine models blocks prostate cancer metastasis (Min et al., "An oncogene-tumor suppressor cascade drives metastatic prostate cancer by coordinately activating Ras and nuclear factor- kappaB," Nat Med. 2010 Mar; 16(3):286-94). EZH2 overexpression is associated with aggressiveness of certain cancers such as breast cancer (Kleer et al., Proc. Nat. Acad. Sci. USA 100:11606-11611, 2003). Recent studies also suggest that prostate cancer specific oncogenic fusion gene TMPRSS2-ERG induces repressive epigenetic programs via direct activation of EZH2 (Yu et al., "An Integrated Network of Androgen Receptor, Polycomb, and TMPRSS2-ERG Gene Fusions in Prostate Cancer Progression," Cancer Cell. 2010 May 18;17(5):443-454).

(R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, represented by the following structure: 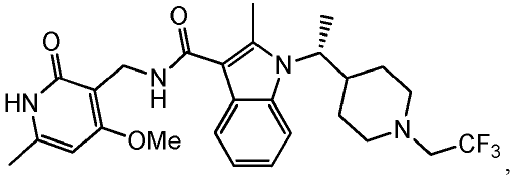 is an effective inhibitor of Enhancer of Zeste Homolog 2 (EZH2) and is useful in treating a variety of conditions associated with methyl modifying enzyme, such as, e.g., in treating proliferative disorders such as cancer. See e.g., U.S. Patent No. 9,085,583 incorporated herein by reference. Also, Vaswani et al. (Journal of Medicinal Chemistry, 2016, 59, 9928-9941, DOI: 10.1021/acs.jmedchem.6b01315) identified (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide as inhibitor of EZH2. Given the therapeutic benefits associated with this compound, a need exists to develop formulations comprising (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide that provide high absorption.

### SUMMARY OF THE INVENTION

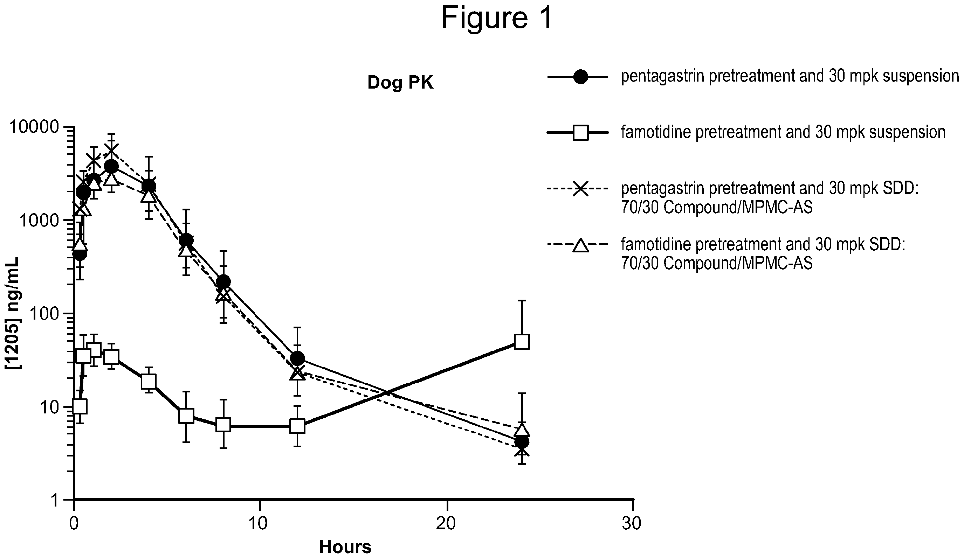

It has now been discovered that (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide has reduced absorption above pH 2.5. See e.g., **Figure 1** showing decreased exposure of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide in dogs following pre-treatment with famotidine.

For healthy subjects not needing treatment for a proliferative disorder, or for those subjects who are not presently taking any medications, this pH dependent characteristic may not present a concern. This is because the gastric environment (ignoring changes associated with the consumption of food and/or changes in digestion) would presumably be at or below the pH level for maximal absorption. However, in cases where subjects are taking medications such as reducers of gastric acid production e.g., proton pump inhibitors (e.g., omeprazole, lansoprazole, esomeprazole, etc.) or H₂ receptor antagonists (e.g., cimetidine, ranitidine, famotidine, etc.), gastric pH levels can rise to pH 4 or higher. See e.g., Aliment Pharmacol Ther. 2011; 34(11):1269-1281. In addition, even in the absence of gastric acid reducers, gastric pH levels can elevate to pH 4-5 simply from the consumption of food and digestion processes. See e.g., The Journal of Clinical Investigation Volume 52 March 1973 645-657. Such a rise in pH, regardless of the cause, would result in a suspension of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide in the stomach. Because (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide has now been found to have reduced bioavailability at these elevated pH levels, treatment would be presumably less effective.

The challenging problem posed by the above discovery was how to avoid a reduction in the bioavailability of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and, as a result, avoid an overall decrease in therapeutic effect, in instances where the gastric environment is above pH 2.5.

Provided herein are solid dispersions of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof; and a pharmaceutically acceptable polymer. These dispersions unexpectedly solve the problem associated with reduced absorption of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide at pH levels above 2.5. See e.g.,. **Figure 1****,** where the pharmacokinetic profile of a solid dispersion of amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide in dogs remained unchanged even after pre-treatment with the known histamine H2-receptor antagonist famotidine (i.e., a reducer of stomach acid) and the known stimulator of gastric acid, pentagastrin.

An additional advantage of the solid dispersions provided herein is that they have high glass transition temperatures. This decreases the risk of conversation and/or crystallization of the dispersed amorphous form at relatively high (above 100 °C) temperatures.

Also provided herein are pharmaceutical compositions (e.g., a tablet) comprising the solid dispersions of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, methods for their manufacture, and uses thereof in treating a variety of conditions such as, e.g., in treating proliferative disorders such as cancer.

### BRIEF DESCRIPTION OF THE FIGURES

**Figure 1** illustrates the *in vivo* pharmacokinetic profile in dogs of a solid dispersion comprising 70 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and HPMC-AS with comparison to a suspension of crystalline (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide after pre-treatment with pentagastrin or famotidine.

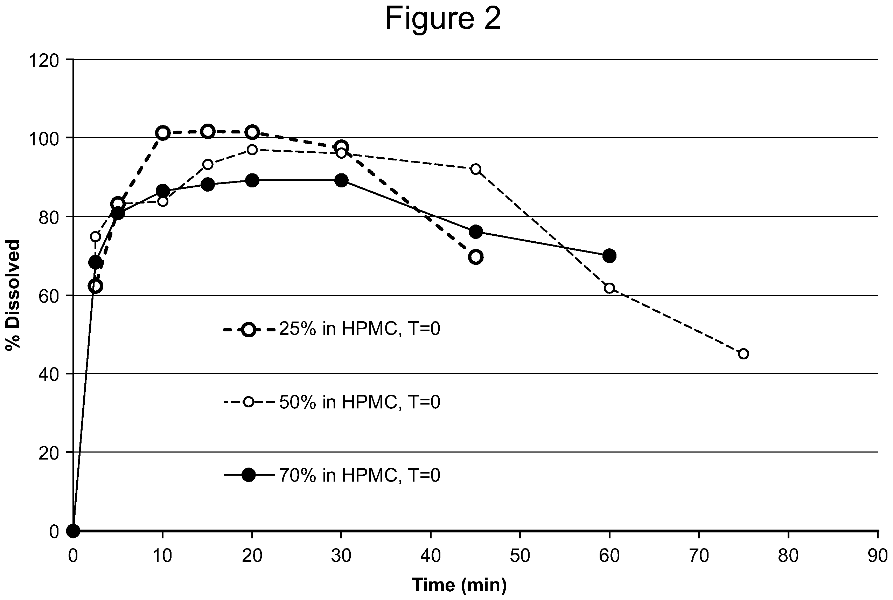

**Figure 2** illustrates the dissolution profile of an exemplary solid dispersion comprising 25 wt%, 50 wt%, and 70% (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and hydroxypropyl methylcellulose (HPMC).

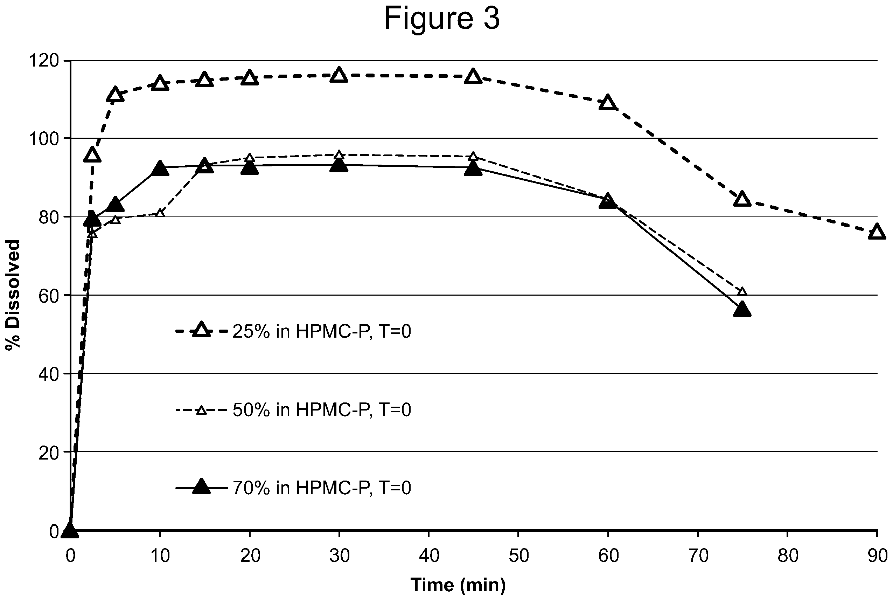

**Figure 3** illustrates the dissolution profile of an exemplary solid dispersion comprising 25 wt%, 50 wt%, and 70% (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and hypromellose phthalate (HPMC-P).

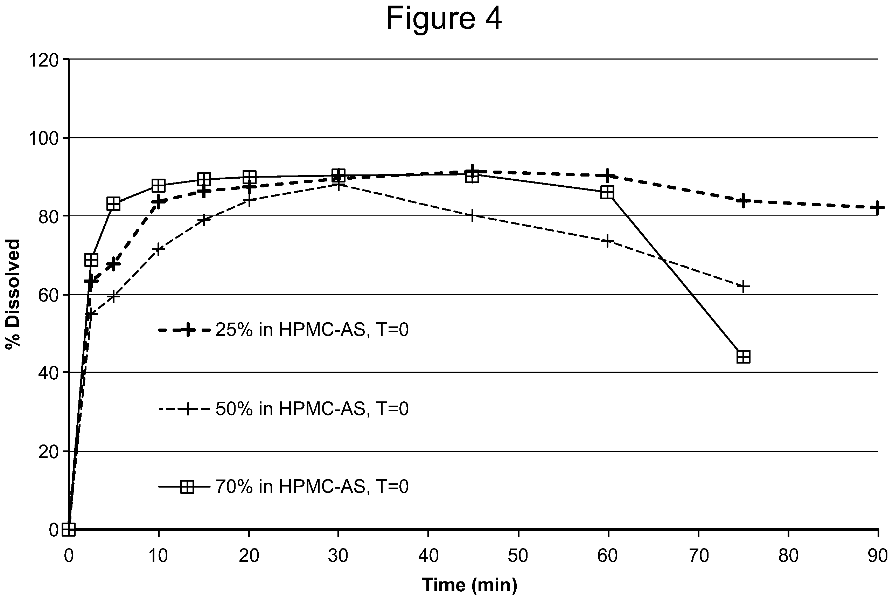

**Figure 4** illustrates the dissolution profile of an exemplary solid dispersion comprising 25 wt%, 50 wt%, and 70% (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and hypromellose acetate succinate (HPMC-AS).

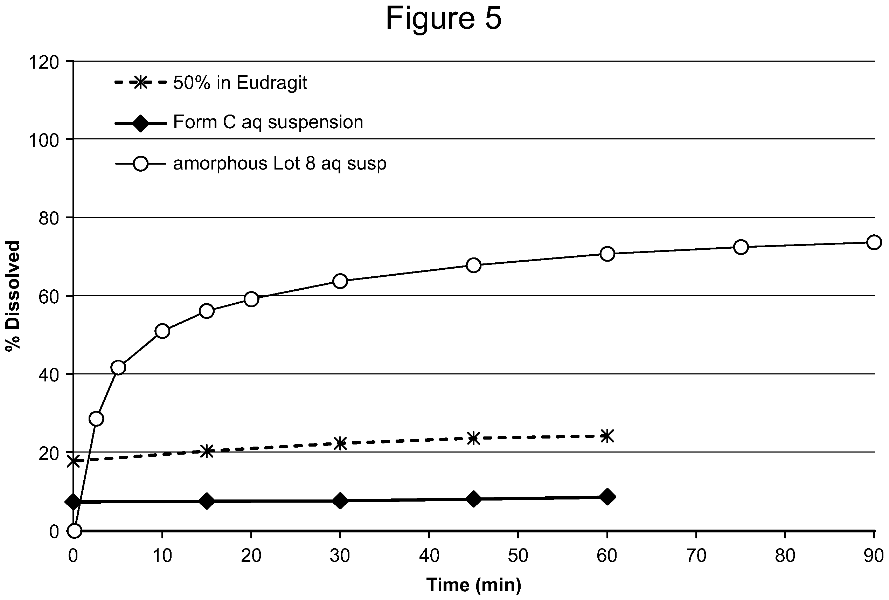

**Figure 5** illustrates the dissolution profile of an exemplary solid dispersion comprising 50 wt% (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and Eudragit L100-55.

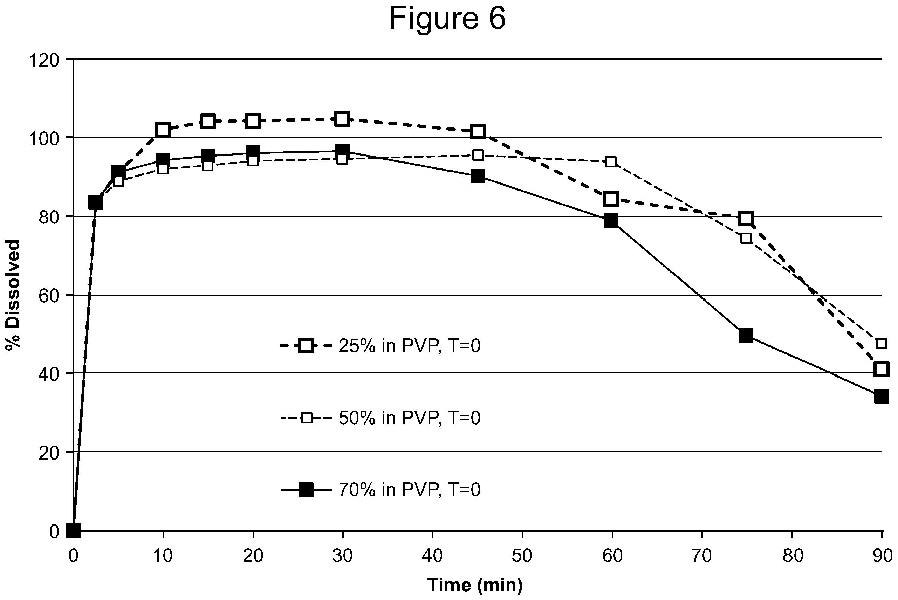

**Figure 6** illustrates the dissolution profile of an exemplary solid dispersion comprising 25 wt%, 50 wt%, and 70% (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and polyvinylpyrrolidone (PVP).

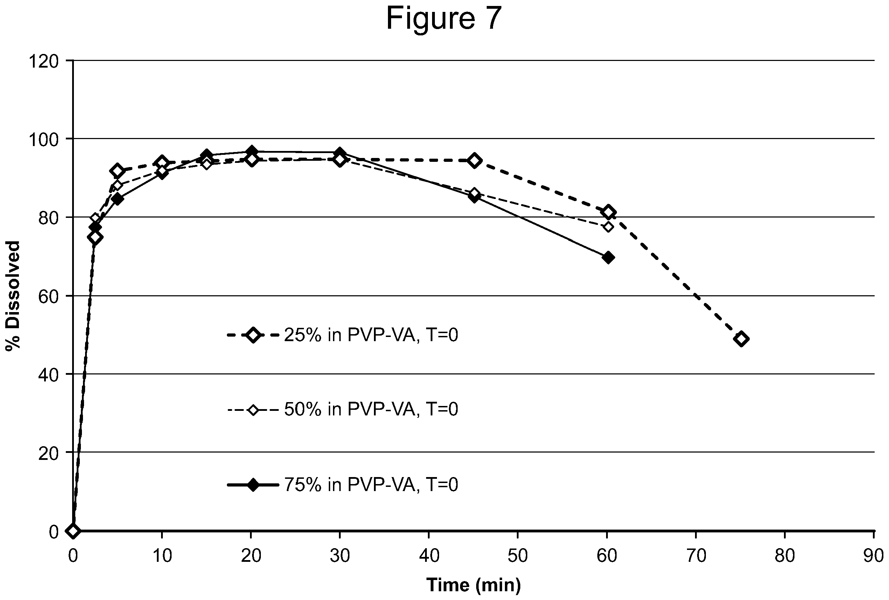

**Figure 7** illustrates the dissolution profile of an exemplary solid dispersion comprising 25 wt%, 50 wt%, and 70% (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and PVP-vinyl acetate (PVP-VA).

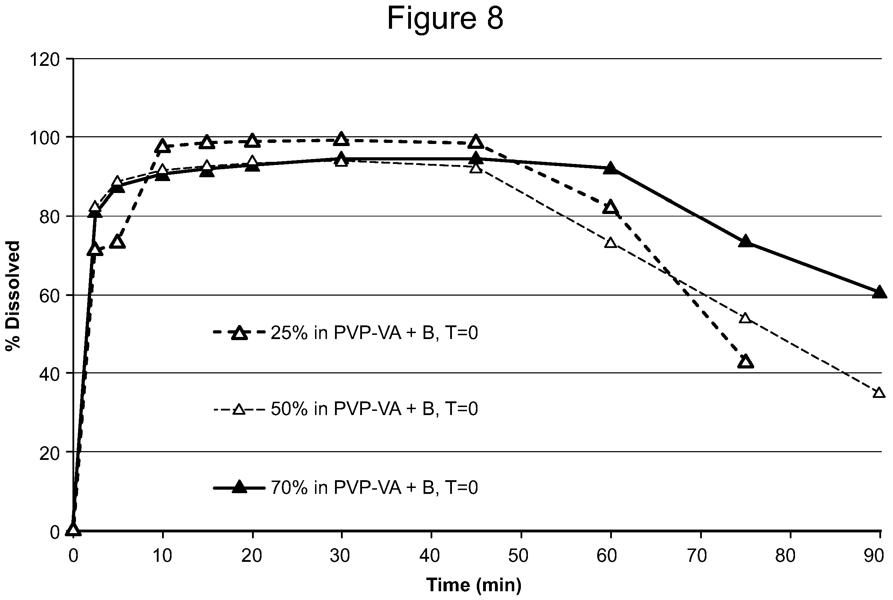

**Figure 8** illustrates the dissolution profile of an exemplary solid dispersion comprising 25 wt%, 50 wt%, and 70% (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and PVP-VA sprayed with sodium bicarbonate.

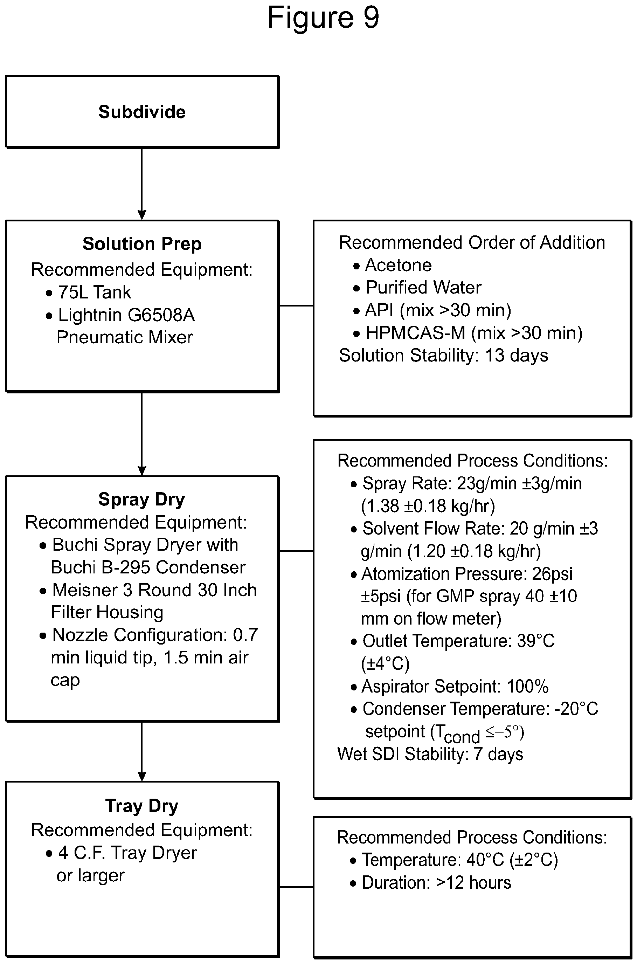

**Figure 9** illustrates an exemplary process for manufacturing a spray-dried dispersion comprising 70 wt% (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and 30 wt% HPMC-AS-M.

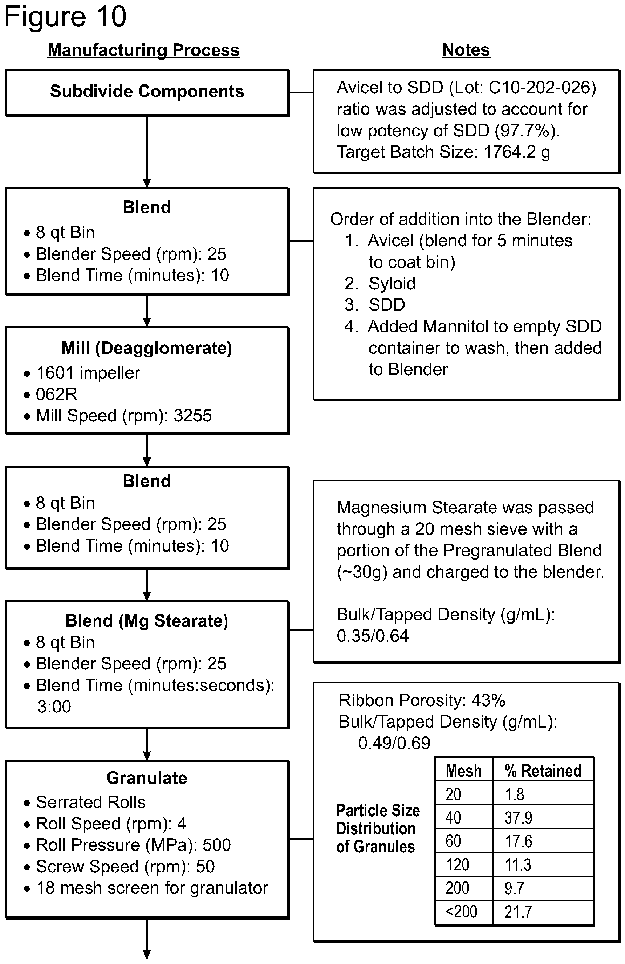

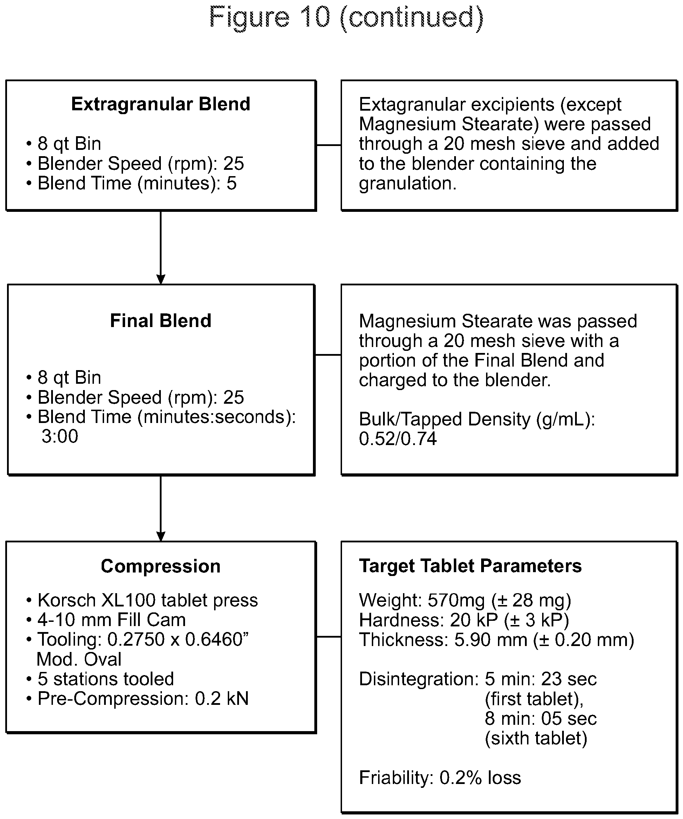

**Figure 10** illustrates an exemplary process for manufacturing a tablet dosage form comprising a solid dispersion comprising 70 wt% (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and 30 wt% HPMC-AS-M.

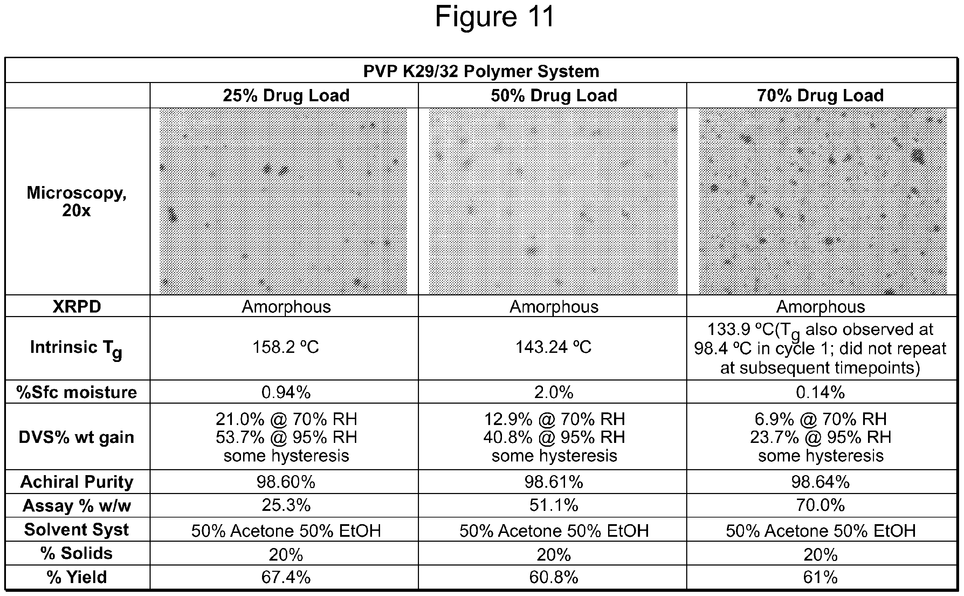

**Figure 11** shows physical characteristics for a solid dispersion comprising 25 wt%, 50 wt%, and 70 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and PVP.

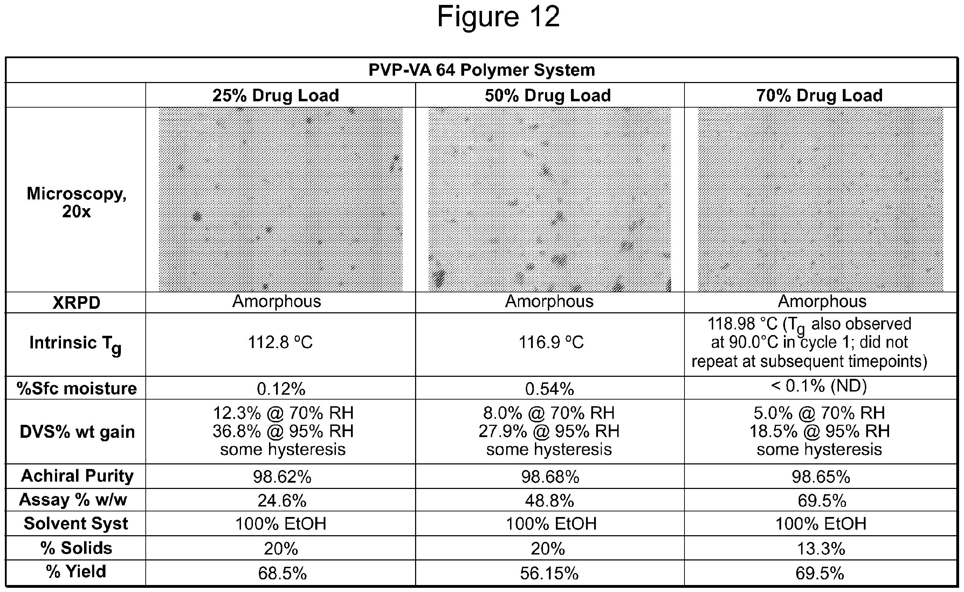

**Figure 12** shows physical characteristics for a solid dispersion comprising 25 wt%, 50 wt%, and 70 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and PVP-VA.

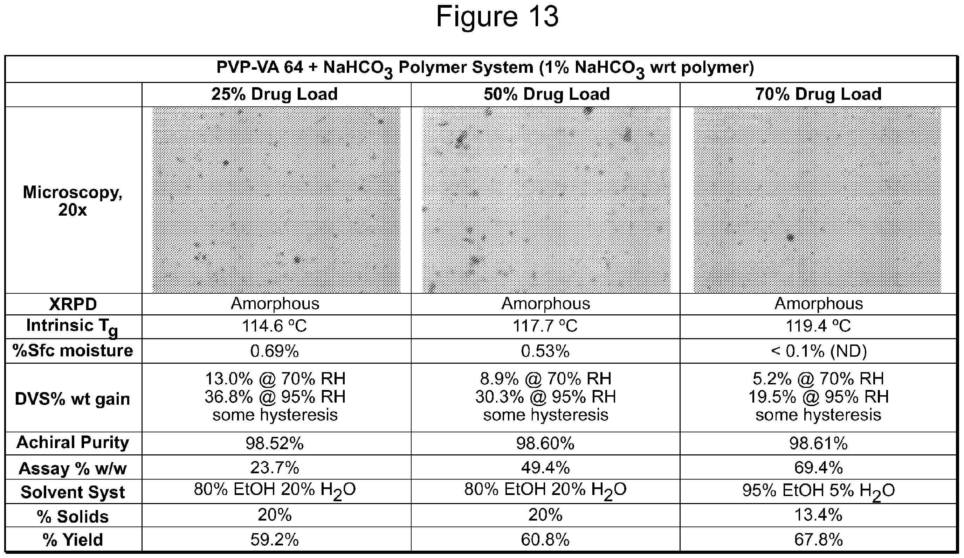

**Figure 13** shows physical characteristics for a solid dispersion comprising 25 wt%, 50 wt%, and 70 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and PVP-VA sprayed with sodium bicarbonate.

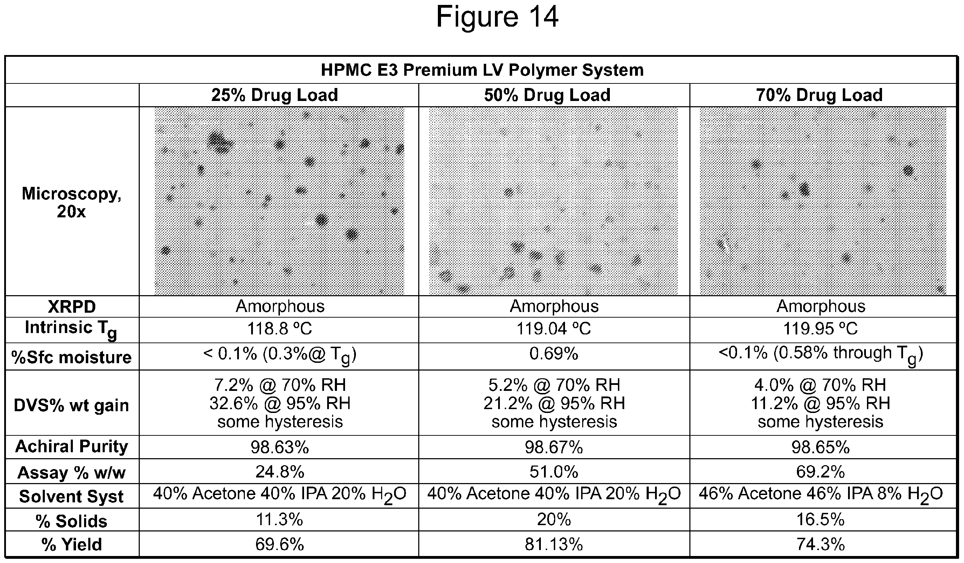

**Figure 14** shows physical characteristics for a solid dispersion comprising 25 wt%, 50 wt%, and 70 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and HPMC.

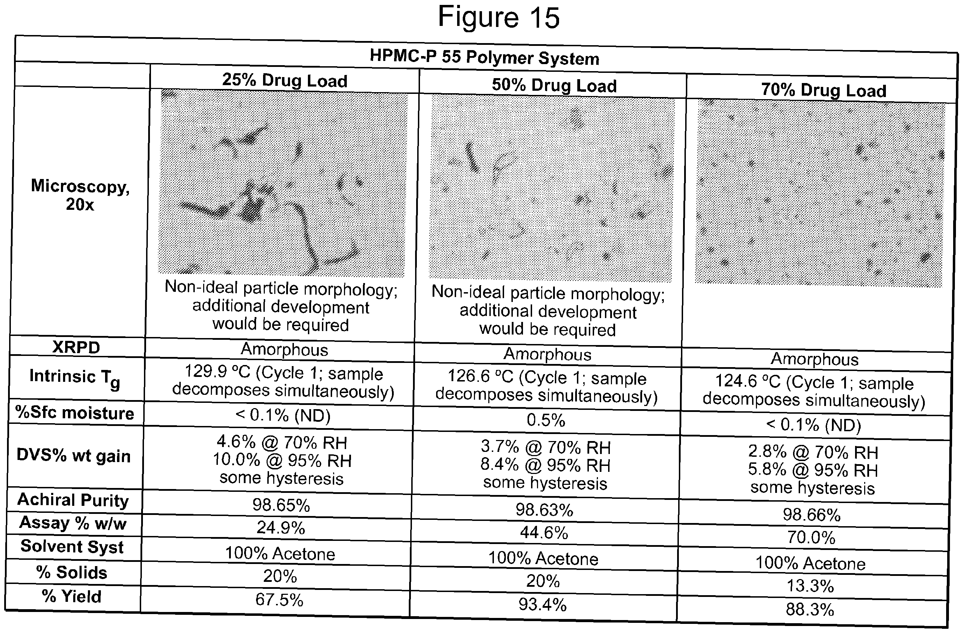

**Figure 15** shows physical characteristics for a solid dispersion comprising 25 wt%, 50 wt%, and 70 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and HPMC-P.

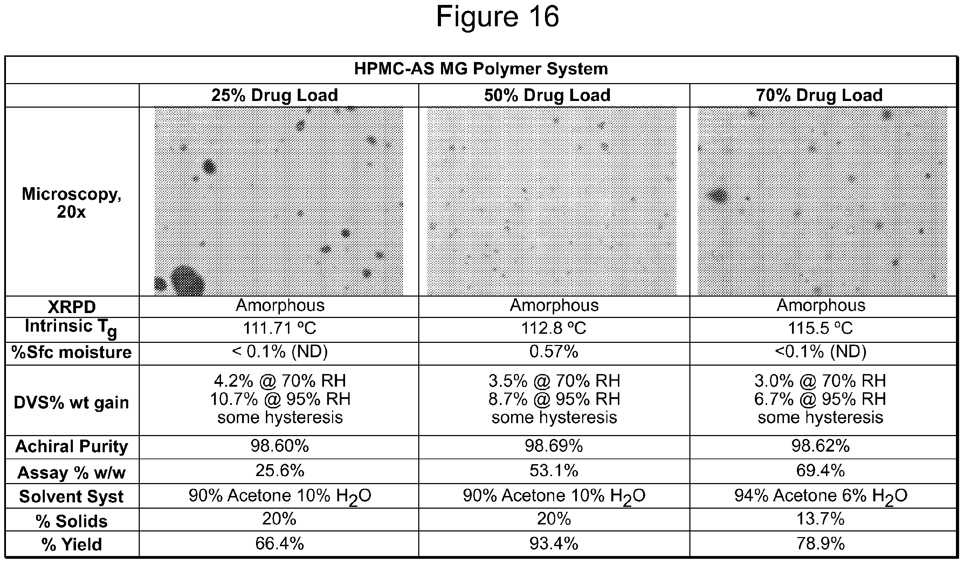

**Figure 16** shows physical characteristics for a solid dispersion comprising 25 wt%, 50 wt%, and 70 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and HPMC-AS.

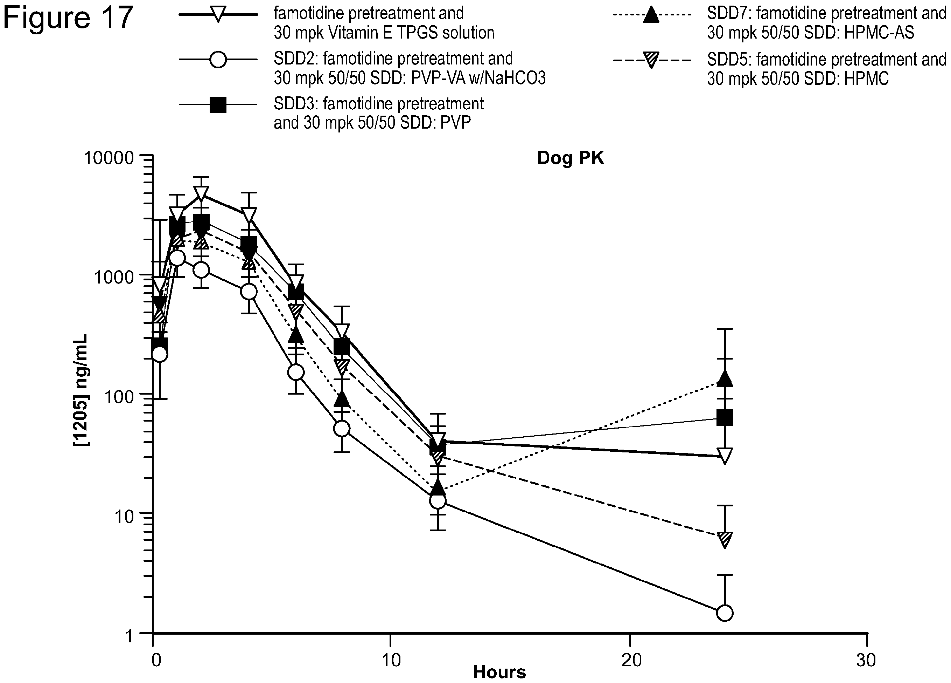

**Figure 17** shows pharmacokinetic studies in famotidine treated dogs using solid dispersions comprising 50 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and PVP, PVP-PA with NaHCO₃, HPMC, and HPMC-AS polymer systems.

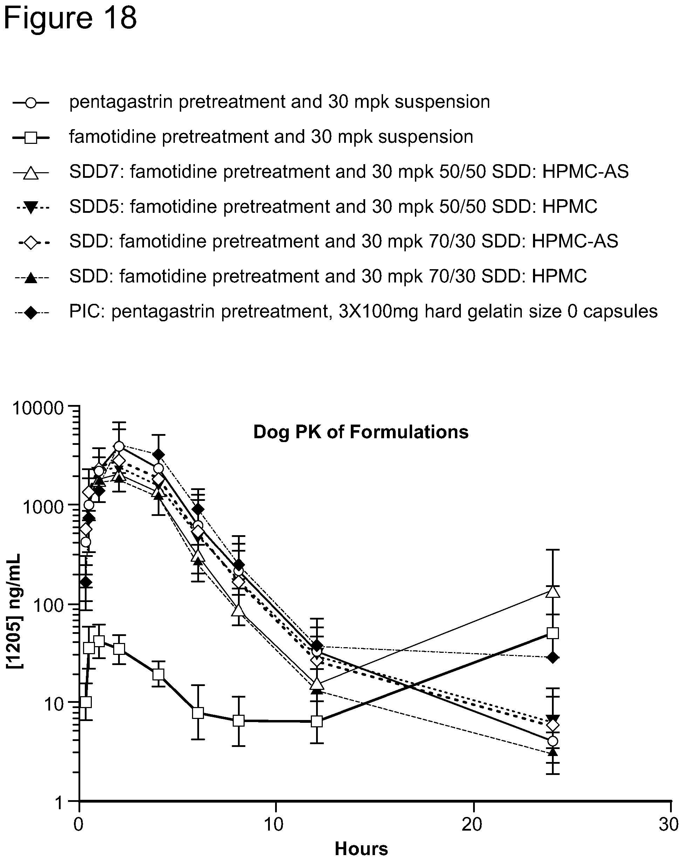

**Figure 18** shows pharmacokinetic studies in famotidine and pentagastrin treated dogs using solid dispersions comprising 50 wt% or 70 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and HPMC, and HPMC-AS polymer systems.

### DETAILED DESCRIPTION

It has now been found that solid dispersions comprising amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide have high absorption across a variety of pH levels. See e.g., **Figure 1****.**

### Definitions

The term "solid dispersion" as used herein refers to a dispersion of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide) and an inert carrier matrix at a solid state, i.e., amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide) is homologously mixed with an inert carrier. The inert matrix is generally hydrophilic (e.g., a polymer) and may be crystalline or amorphous. It will be understood that it is not necessarily the preparation method that governs the properties of the solid dispersion, but rather the molecular arrangement of the contents of the dispersion. Thus, absent an expression to do so, or an incorporation of process restrictions, solid dispersions are not to be limited by the process to which they are made.

The term "amorphous" means a solid that is present in a non-crystalline state or form. Amorphous solids are disordered arrangements of molecules and therefore possess no distinguishable crystal lattice or unit cell and consequently have no definable long range ordering. Solid state ordering of solids may be determined by standard techniques known in the art, e.g., by X-ray powder diffraction (XRPD) or differential scanning calorimetry (DSC). Amorphous solids can also be differentiated from crystalline solids e.g., by birefringence using polarized light microscopy.

"(R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide" refers to a compound of the following structure: 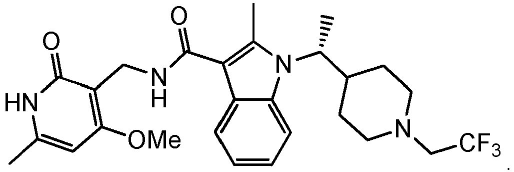 Unless otherwise indicated, (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide) is present in the disclosed dispersions as an amorphous form. Thus, when referring to a disclosed dispersion both "amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide)" and "(R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide)" are used interchangeably and mean the amorphous form. The synthesis of the amorphous form of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide) is described in U.S. Patent No. 9,085,583, the contents of which are incorporated herein by reference.

Pharmaceutically acceptable salts are art-recognized and include e.g., relatively non-toxic inorganic and organic acid addition salts, or inorganic or organic base addition salts that are suitable for human consumption. Examples of such salts include, but are not limited to, sodium, potassium, calcium, magnesium, acetate, benzoate, bicarbonate, carbonate, citrate, dihydrochloride, gluconate, glutamate, hydrochloride, and tartrate.

The term "pharmaceutically acceptable carrier" refers to a non-toxic carrier, adjuvant, or vehicle that does not adversely affect the pharmacological activity of the compound with which it is formulated, and which is also safe for human use. Pharmaceutically acceptable carriers, adjuvants or vehicles that may be used in the compositions of this disclosure include, but are not limited to, ion exchangers, alumina, aluminum stearate, magnesium stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, dicalcium phosphate, potassium hydrogen phosphate, sodium chloride, zinc salts, colloidal silica, magnesium trisilicate, polyvinyl pyrrolidone, polyvinylpyrrolidone-vinyl acetate, cellulose-based substances (e.g., microcrystalline cellulose, hydroxypropyl methylcellulose, hydroxypropyl methylcellulose acetate succinate, hydroxypropyl methylcellulose Phthalate), starch, lactose monohydrate, mannitol, sodium lauryl sulfate, and crosscarmellose sodium, polyethylene glycol, sodium carboxymethylcellulose, polyacrylates, polymethacrylate, waxes, polyethylene-polyoxypropylene-block polymers, polyethylene glycol and wool fat.

As used herein, the terms "treatment," "treat," and "treating" refer to reversing, alleviating, or inhibiting the progress of a disease or disorder, or one or more symptoms thereof, as described herein.

The term "spray drying" or "spray dried" refers to processes which involve the atomization of a suspension or solution of a drug into small droplets and rapidly removing solvent in a processing chamber where there is a strong driving force for the evaporation (e.g., hot dry gas or partial vacuum, or combinations thereof).

### Compositions

In one embodiment, the present disclosure provides a solid dispersion comprising amorphous N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, represented by the following structure: 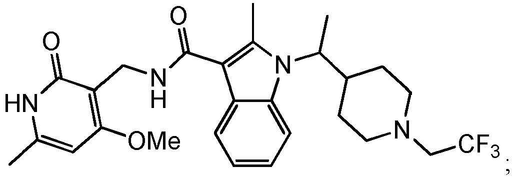 or a pharmaceutically acceptable salt thereof; and a pharmaceutically acceptable polymer.

In another embodiment, the present disclosure provides a solid dispersion comprising amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide; or a pharmaceutically acceptable salt thereof; and a pharmaceutically acceptable polymer.

Pharmaceutically acceptable polymers include those polymers which are suitable for human consumption. Examples include cellulose-based polymers (e.g., methyl cellulose, ethyl cellulose, carboxymethyl cellulose, hydroxyethyl cellulose, hydroxypropyl cellulose, hydroxypropyl methylcellulose (HPMC), hypromellose phthalate (HPMC-P), hypromellose acetate succinate (HPMC-AS), etc.), polyethylene glycol, polyethylene glycol vinyl alcohol polymers, polyethylene oxide, polyvinyl pyrrolidone, and polyacrylate or polymethacrylate esters containing anionic and cationic functionalities. In one embodiment, the pharmaceutically acceptable polymers in the solid dispersions herein are selected from polyvinylpyrrolidone (PVP), polyvinylpyrrolidone/vinyl acetate copolymer (PVP-VA), hydroxypropyl methylcellulose (HPMC), hypromellose phthalate (HPMC-P), and hypromellose acetate succinate (HPMC-AS). Polyvinylpyrrolidones may have molecular weight averages between 2,000 and 3,000 (e.g., Kollidon ®12 PF), between 7,000 and 11,000 (e.g., Kollidon® 17 PF) between 28,000 and 34,000 (e.g., Kollidon® 25), between 44,000 and 54,000 (e.g., Kollidon® 30), and between 1,000,000 and 1,500,000 (e.g., Kollidon® 90 or Kollidon® 90F).

In one embodiment, the pharmaceutically acceptable polymer present in the described dispersions is HPMC-P or HPMC-AS. Different grades of HPMC-P and HPMC-AS may also be used. For example, HPMC-P grade 50 (HP-50) can be used, which has a nominal phthalyl content of 24 wt%, a pH solubility of ≥ 5, and a labeled viscosity (cSt) of 55. Alternatively, HPMC-P grade 55 (HP-55) can be used, which has a nominal phthalyl content of 31 wt%, a pH solubility of ≥ 5.5, and a labeled viscosity (cSt) of 40. Or, in another alternative, HPMC-P grade 55S (HP-5 5 S) can be used, which has a nominal phthalyl content of 31 wt%, a pH solubility of ≥ 5.5, and a labeled viscosity (cSt) of 170. In other alternatives, HPMC-AS grades L, M, or H can be used. HPMC-AS grade L (HPMC-AS-L) has an acetyl content of 5-9 wt%, a succinoyl content of 14-18 wt%, a methoxyl content of 20-24 wt%, and a hydropropoxy content of 5-9 wt%. HPMC-AS grade M (HPMC-AS-M) has an acetyl content of 7-11 wt%, an succinoyl content of 10-14 wt%, a methoxyl content of 21-25 wt%, and a hydropropoxy content of 5-9 wt%. HPMC-AS grade H (HPMC-AS-H) has an acetyl content of 10-14 wt%, an succinoyl content of 4-8 wt%, a methoxyl content of 22-26 wt%, and a hydropropoxy content of 6-10 wt%. Each of HPMC-AS-L, HPMC-AS-M, and HPMC-AS-H can be of fine (an average particle size of 5 µm) or granular (an average particle size of 1 mm) particle sizes. In one embodiment, the pharmaceutically acceptable polymer present in the described dispersions is HPMC-AS-M.

The amount of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and pharmaceutically acceptable polymer in the solid dispersion can vary. Acceptable weight ratios include e.g., from 10 wt% to 90 wt% compound and from 90 wt% to 10 wt% pharmaceutically acceptable polymer, and all ranges in between, e.g., from 15 wt% to 85 wt%, from 20 wt% to 80 wt%, from 25 wt% to 75 wt%, from 30 wt% to 70 wt%, from 35 wt% to 65 wt%, from 40 wt% to 60 wt%, from 45 wt% to 55 wt%, from 50 wt% to 90 wt%, from 60 wt% to 80 wt%, from 65% to 75%, 70 wt% and 50 wt% compound and from 85 wt% to 15 wt%, from 80 wt% to 20 wt%, from 75 wt% to 25 wt%, from 70 wt% to 30 wt%, from 65 wt% to 35 wt%, from 60 wt% to 40 wt%, from 55 wt% to 45 wt%, from 10 wt% to 60 wt%, from 20 wt% to 40 wt%, from 25 wt% to 35 wt%, 30 wt%, and 50 wt% pharmaceutically acceptable polymer.

In one embodiment the weight ratio of the pharmaceutically acceptable polymer to (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide is 50:50 wt% or 30:70 wt%. In another embodiment the solid dispersions described herein comprise (R)-N-((4-methoxy-6-methyl-2-oxo-1 ,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof; and HPMC-AS or HPMC-AS-M, wherein the weight ratio of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide to HPMC-AS or HPMC-AS-M is 50:50 or 70:30 wt%.

As described above, a further advantage exits in the high Tg inflection point of the disclosed solid dispersions. Thus, in one embodiment, the disclosed solid dispersions exhibit a Tg of greater than 100 °C. In another embodiment, the disclosed solid dispersion exhibits a Tg inflection point ranging from 100 °C to 160 °C, from 105 °C to 130 °C, or from 110 °C to 120 °C. In another embodiment, the solid dispersions described herein exhibit a Tg inflection point ranging from 113 °C to 118 °C. In another embodiment, the solid dispersions described herein exhibit a Tg inflection point of 115 °C.

In one embodiment, the solid dispersions described herein exhibit a Tg ranging from 110 °C to 120 °C and comprise HPMC-AS or HPMC-AS-M as the pharmaceutically acceptable polymer and amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof. In another embodiment, the solid dispersions described herein exhibit a Tg of 115 °C and comprise HPMC-AS or HPMC-AS-M as the pharmaceutically acceptable polymer and amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof.

In one embodiment, the solid dispersions described herein exhibit a Tg ranging from 110 °C to 120 °C and comprise from 20 wt% to 40 wt% HPMC-AS or from 20 wt% to 40 wt% HPMC-AS-M as the pharmaceutically acceptable polymer and from 60 wt% to 80 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof. In another embodiment, the solid dispersions described herein exhibit a Tg of 115 °C and comprise from 20 wt% to 40 wt% HPMC-AS or from 20 wt% to 40 wt% HPMC-AS-M as the pharmaceutically acceptable polymer and from 60 wt% to 80 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof.

In one embodiment, the solid dispersions described herein exhibit a Tg ranging from 110 °C to 120 °C and comprise from 25 wt% to 35 wt% HPMC-AS or from 25 wt% to 35 wt% HPMC-AS-M as the pharmaceutically acceptable polymer and from 65 wt% to 75 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof. In another embodiment, the solid dispersions described herein exhibit a Tg of 115 °C and comprise from 25 wt% to 35 wt% HPMC-AS or from 25 wt% to 35 wt% HPMC-AS-M as the pharmaceutically acceptable polymer and from 65 wt% to 75 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof.

In one embodiment, the solid dispersions described herein exhibit a Tg ranging from 110 °C to 120 °C and comprise HPMC-AS or HPMC-AS-M as the pharmaceutically acceptable polymer and amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof, wherein the weight ratio of the pharmaceutically acceptable polymer to (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide is 50:50 wt% or 30:70 wt%. In another embodiment, the solid dispersions described herein exhibit a Tg of 115 °C and comprise HPMC-AS or HPMC-AS-M as the pharmaceutically acceptable polymer and amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof, wherein the weight ratio of the pharmaceutically acceptable polymer to (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide is 50:50 wt% or 30:70 wt%.

In one embodiment, the solid dispersions herein comprise an average particle size (Dv₅₀) ranging from 5 µM to 20 µM e.g., from 10 µM to 12 µM. Thus, for example, in one embodiment, the solid dispersions herein comprise a pharmaceutically acceptable polymer as described in the preceding paragraphs together with an amount of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide as defined in the preceding paragraphs, wherein the solid dispersion comprises an average particle size (Dv₅₀) ranging from 5 µM to 20 µM (e.g., from 10 µM to 12 µM) and exhibits a Tg inflection point ranging from 100 °C to 160 °C, from 105 °C to 130 °C, or from 110 °C to 120 °C (e.g., ranging 113 °C to 118 °C or at 115 °C).

In one embodiment, the solid dispersions described herein exhibit a Tg ranging from 110 °C to 120 °C, comprise an average particle size (Dv₅₀) ranging from 5 µM to 20 µM (e.g., from 10 µM to 12 µM), and comprise HPMC-AS or HPMC-AS-M as the pharmaceutically acceptable polymer and amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof. In another embodiment, the solid dispersions described herein exhibit a Tg of 115 °C, comprise an average particle size (Dv₅₀) ranging from 5 µM to 20 µM (e.g., from 10 µM to 12 µM), and comprise HPMC-AS or HPMC-AS-M as the pharmaceutically acceptable polymer and amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof.

In one embodiment, the solid dispersions described herein exhibit a Tg ranging from 110 °C to 120 °C, comprise an average particle size (Dv₅₀) ranging from 5 µM to 20 µM (e.g., from 10 µM to 12 µM), and comprise from 20 wt% to 40 wt% HPMC-AS or from 20 wt% to 40 wt% HPMC-AS-M as the pharmaceutically acceptable polymer and from 60 wt% to 80 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof. In another embodiment, the solid dispersions described herein exhibit a Tg of 115 °C, comprise an average particle size (Dv₅₀) ranging from 5 µM to 20 µM (e.g., from 10 µM to 12 µM), and comprise from 20 wt% to 40 wt% HPMC-AS or from 20 wt% to 40 wt% HPMC-AS-M as the pharmaceutically acceptable polymer and from 60 wt% to 80 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof.

In one embodiment, the solid dispersions described herein exhibit a Tg ranging from 110 °C to 120 °C, comprise an average particle size (Dv₅₀) ranging from 5 µM to 20 µM (e.g., from 10 µM to 12 µM), and comprise from 25 wt% to 35 wt% HPMC-AS or from 25 wt% to 35 wt% HPMC-AS-M as the pharmaceutically acceptable polymer and from 65 wt% to 75 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof. In another embodiment, the solid dispersions described herein exhibit a Tg of 115 °C, comprise an average particle size (Dv₅₀) ranging from 5 µM to 20 µM (e.g., from 10 µM to 12 µM), and comprise from 25 wt% to 35 wt% HPMC-AS or from 25 wt% to 35 wt% HPMC-AS-M as the pharmaceutically acceptable polymer and from 65 wt% to 75 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof.

In one embodiment, the solid dispersions described herein exhibit a Tg ranging from 110 °C to 120 °C, comprise an average particle size (Dv₅₀) ranging from 5 µM to 20 µM (e.g., from 10 µM to 12 µM), and comprise HPMC-AS or HPMC-AS-M as the pharmaceutically acceptable polymer and amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof, wherein the weight ratio of the pharmaceutically acceptable polymer to (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide is 50:50 wt% or 30:70 wt%. In another embodiment, the solid dispersions described herein exhibit a Tg of 115 °C, comprise an average particle size (Dv₅₀) ranging from 5 µM to 20 µM (e.g., from 10 µM to 12 µM), and comprise HPMC-AS or HPMC-AS-M as the pharmaceutically acceptable polymer and amorphous (R)-N-((4-methoxy-6-methyl-2-oxo-1 ,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof, wherein the weight ratio of the pharmaceutically acceptable polymer to (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide is 50:50 wt% or 30:70 wt%.

In one embodiment, the solid dispersions herein comprise a bulk density ranging 0.2 g/mL to 0.3 g/mL. Thus, for example, in one embodiment, the solid dispersions herein comprise a pharmaceutically acceptable polymer as described in the preceding paragraphs together with an amount of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide as defined in the preceding paragraphs, wherein the solid dispersion comprises an average particle size (Dv₅₀) ranging from 5 µM to 20 µM (e.g., from 10 µM to 12 µM), exhibits a Tg inflection point ranging from 100 °C to 160 °C, from 105 °C to 130 °C, or from 110 °C to 120 °C (e.g., ranging 113 °C to 118 °C or at 115 °C), and comprises a bulk density ranging 0.2 g/mL to 0.3 g/mL.

In one embodiment, the solid dispersions described herein exhibit a Tg ranging from 110 °C to 120 °C, comprise a bulk density ranging 0.2 g/mL to 0.3 g/mL, comprise an average particle size (Dv₅₀) ranging from 5 µM to 20 µM (e.g., from 10 µM to 12 µM), and comprise HPMC-P or HPMC-AS as the pharmaceutically acceptable polymer and amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof. In another embodiment, the solid dispersions described herein exhibit a Tg of 115 °C, comprise a bulk density ranging 0.2 g/mL to 0.3 g/mL, comprise an average particle size (Dv₅₀) ranging from 5 µM to 20 µM (e.g., from 10 µM to 12 µM), and comprise HPMC-P or HPMC-AS as the pharmaceutically acceptable polymer and amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof.

In one embodiment, the solid dispersions described herein exhibit a Tg ranging from 110 °C to 120 °C, comprise a bulk density ranging 0.2 g/mL to 0.3 g/mL, comprise an average particle size (Dv₅₀) ranging from 5 µM to 20 µM (e.g., from 10 µM to 12 µM), and comprise from 20 wt% to 40 wt% HPMC-P or from 20 wt% to 40 wt% HPMC-AS as the pharmaceutically acceptable polymer and from 60 wt% to 80 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof. In another embodiment, the solid dispersions described herein exhibit a Tg of 115 °C, comprise a bulk density ranging 0.2 g/mL to 0.3 g/mL, comprise an average particle size (Dv₅₀) ranging from 5 µM to 20 µM (e.g., from 10 µM to 12 µM), and comprise from 20 wt% to 40 wt% HPMC-P or from 20 wt% to 40 wt% HPMC-AS as the pharmaceutically acceptable polymer and from 60 wt% to 80 wt% amorphous (R)-N-((4-methoxy-6-methyl-2-oxo-1 ,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof.

In one embodiment, the solid dispersions described herein exhibit a Tg ranging from 110 °C to 120 °C, comprise a bulk density ranging 0.2 g/mL to 0.3 g/mL, comprise an average particle size (Dv₅₀) ranging from 5 µM to 20 µM (e.g., from 10 µM to 12 µM), and comprise from 25 wt% to 35 wt% HPMC-P or from 25 wt% to 35 wt% HPMC-AS as the pharmaceutically acceptable polymer and from 65 wt% to 75 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof. In another embodiment, the solid dispersions described herein exhibit a Tg of 115 °C, comprise a bulk density ranging 0.2 g/mL to 0.3 g/mL, comprise an average particle size (Dv₅₀) ranging from 5 µM to 20 µM (e.g., from 10 µM to 12 µM), and comprise from 25 wt% to 35 wt% HPMC-P or from 25 wt% to 35 wt% HPMC-AS as the pharmaceutically acceptable polymer and from 65 wt% to 75 wt% amorphous (R)-N-((4-methoxy-6-methyl-2-oxo-1 ,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof.

In one embodiment, the solid dispersions described herein exhibit a Tg ranging from 110 °C to 120 °C, comprise a bulk density ranging 0.2 g/mL to 0.3 g/mL, comprise an average particle size (Dv₅₀) ranging from 5 µM to 20 µM (e.g., from 10 µM to 12 µM), and comprise HPMC-P or HPMC-AS as the pharmaceutically acceptable polymer and amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof, wherein the weight ratio of the pharmaceutically acceptable polymer to (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide is 50:50 wt% or 30:70 wt%. In another embodiment, the solid dispersions described herein exhibit a Tg of 115 °C, comprise a bulk density ranging 0.2 g/mL to 0.3 g/mL, comprise an average particle size (Dv₅₀) ranging from 5 µM to 20 µM (e.g., from 10 µM to 12 µM), and comprise HPMC-P or HPMC-AS as the pharmaceutically acceptable polymer and amorphous (R)-N-((4-methoxy-6-methyl-2-oxo-1 ,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof, wherein the weight ratio of the pharmaceutically acceptable polymer to (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide is 50:50 wt% or 30:70 wt%.

In one embodiment, the solid dispersions described herein exhibit a Tg ranging from 110 °C to 120 °C, comprise a bulk density ranging 0.2 g/mL to 0.3 g/mL, comprise an average particle size (Dv₅₀) ranging from 5 µM to 20 µM (e.g., from 10 µM to 12 µM), and comprise HPMC-AS or HPMC-AS-M as the pharmaceutically acceptable polymer and amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof. In another embodiment, the solid dispersions described herein exhibit a Tg of 115 °C, comprise a bulk density ranging 0.2 g/mL to 0.3 g/mL, comprise an average particle size (Dv₅₀) ranging from 5 µM to 20 µM (e.g., from 10 µM to 12 µM), and comprise HPMC-AS or HPMC-AS-M as the pharmaceutically acceptable polymer and amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof.

In one embodiment, the solid dispersions described herein exhibit a Tg ranging from 110 °C to 120 °C, comprise a bulk density ranging 0.2 g/mL to 0.3 g/mL, comprise an average particle size (Dv₅₀) ranging from 5 µM to 20 µM (e.g., from 10 µM to 12 µM), and comprise from 20 wt% to 40 wt% HPMC-AS or from 20 wt% to 40 wt% HPMC-AS-M as the pharmaceutically acceptable polymer and from 60 wt% to 80 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof. In another embodiment, the solid dispersions described herein exhibit a Tg of 115 °C, comprise a bulk density ranging 0.2 g/mL to 0.3 g/mL, comprise an average particle size (Dv₅₀) ranging from 5 µM to 20 µM (e.g., from 10 µM to 12 µM), and comprise from 20 wt% to 40 wt% HPMC-AS or from 20 wt% to 40 wt% HPMC-AS-M as the pharmaceutically acceptable polymer and from 60 wt% to 80 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof.

In one embodiment, the solid dispersions described herein exhibit a Tg ranging from 110 °C to 120 °C, comprise a bulk density ranging 0.2 g/mL to 0.3 g/mL, comprise an average particle size (Dv₅₀) ranging from 5 µM to 20 µM (e.g., from 10 µM to 12 µM), and comprise from 25 wt% to 35 wt% HPMC-AS or from 25 wt% to 35 wt% HPMC-AS-M as the pharmaceutically acceptable polymer and from 65 wt% to 75 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof. In another embodiment, the solid dispersions described herein exhibit a Tg of 115 °C, comprise a bulk density ranging 0.2 g/mL to 0.3 g/mL, comprise an average particle size (Dv₅₀) ranging from 5 µM to 20 µM (e.g., from 10 µM to 12 µM), and comprise from 25 wt% to 35 wt% HPMC-AS or from 25 wt% to 35 wt% HPMC-AS-M as the pharmaceutically acceptable polymer and from 65 wt% to 75 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof.

In one embodiment, the solid dispersions described herein exhibit a Tg ranging from 110 °C to 120 °C, comprise a bulk density ranging 0.2 g/mL to 0.3 g/mL, comprise an average particle size (Dv₅₀) ranging from 5 µM to 20 µM (e.g., from 10 µM to 12 µM), and comprise HPMC-AS or HPMC-AS-M as the pharmaceutically acceptable polymer and amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof, wherein the weight ratio of the pharmaceutically acceptable polymer to (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide is 50:50 wt% or 30:70 wt%. In another embodiment, the solid dispersions described herein exhibit a Tg of 115 °C, comprise a bulk density ranging 0.2 g/mL to 0.3 g/mL, comprise an average particle size (Dv₅₀) ranging from 5 µM to 20 µM (e.g., from 10 µM to 12 µM), and comprise HPMC-AS or HPMC-AS-M as the pharmaceutically acceptable polymer and amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof, wherein the weight ratio of the pharmaceutically acceptable polymer to (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide is 50:50 wt% or 30:70 wt%.

### Uses, Formulation and Administration Dosage Forms

According to other embodiments, the present disclosure relates to a method of inhibiting EZH2 using a solid dispersion described herein; and a pharmaceutically acceptable carrier, adjuvant, or vehicle. The amount of solid dispersion in a provided composition is such that is effective to measurably modulate a histone methyl modifying enzyme, or a mutant thereof, in a biological sample or in a patient. In some embodiments, a provided composition is formulated to treat a subject with a proliferative disorder such as cancer.

In some embodiments, the disclosed solid dispersions can be used for treating a subject with a proliferative disorder (such as cancer), wherein the subject is taking a medication which alters gastric pH levels. In one embodiment, the medication which alters gastric pH levels raises gastric pH levels. Medications that alter gastric pH levels include, but are not limited to antacids (e.g., bicarbonates, hydroxides, carbonates) and acid reducers (H2-receptor antagonists or proton pump inhibitors).

In one embodiment, the subject treated by the solid dispersions defined herein has gastric pH levels of 2.5 or higher, e.g., 2.7 or higher or 3.0 or higher. Although it could be, these levels do not necessarily have to be related to or a direct consequence resulting from the consumption of a medication which alters gastric pH levels (such as those listed above). Thus, the subjects defined herein may have gastric pH levels of 2.5 or higher (e.g., 2.7 or higher or 3.0 or higher) naturally. Or such levels could result from food consumption. In one embodiment, however, the gastric pH levels of 2.5 or higher, e.g., 2.7 or higher or 3.0 or higher are a consequence of medication that raises gastric pH, such as e.g., those defined in the preceding paragraph.

Suitable dosage forms that can be used with the solid dispersions herein include, but are not limited to, capsules, tablets, mini-tablets, beads, beadlets, pellets, granules, granulates, and powder. Suitable dosage forms may be coated, for example using an enteric coating. In some embodiments, the solid dispersions are formulated as tablets, caplets, or capsules. In one embodiment, the solid dispersions are formulated as a tablet. In one embodiment, the pharmaceutical composition further includes one or more additives such as disintegrants, lubricants, glidants, binders, and fillers.

Examples of suitable pharmaceutically acceptable lubricants and pharmaceutically acceptable glidants for use with the pharmaceutical compositions herein include, but are not limited to, colloidal silica (e.g., Syloid 244 FP from Grace Davison), magnesium trisilicate, starches, talc, tribasic calcium phosphate, magnesium stearate (e.g., magnesium stearate 2257 from Mallinckrodt), aluminum stearate, calcium stearate, magnesium carbonate, magnesium oxide, polyethylene glycol, powdered cellulose, glyceryl behenate, stearic acid, hydrogenated castor oil, glyceryl monostearate, and sodium stearyl fumarate.

Examples of suitable pharmaceutically acceptable binders for use with the pharmaceutical compositions herein include, but are not limited to starches; celluloses and derivatives thereof, e.g., microcrystalline cellulose (e.g., Avicel PH 102 from FMC Biopolymer), hydroxypropyl cellulose, hydroxyethyl cellulose, crosscarmellose sodium (e.g., Ac-Di-Sol from FMC Biopolymer) and hydroxylpropylmethylcellulose (HPMC, e.g., METHOCEL from Dow Chemical); sucrose, dextrose, corn syrup; polysaccharides; and gelatin.

Examples of suitable pharmaceutically acceptable fillers and pharmaceutically acceptable diluents for use with the pharmaceutical composition include, but are not limited to, confectioner's sugar, compressible sugar, dextrates, dextrin, dextrose, lactose, mannitol (e.g., Parteck M100 from EMD Millipore), microcrystalline cellulose (MCC), powdered cellulose, sorbitol, sucrose, and talc.

In some embodiments, excipients may serve more than one function in the pharmaceutical compositions. For example, fillers or binders may also be disintegrants, glidants, anti-adherents, lubricants, sweeteners and the like.

In one embodiment, the present disclosure provides for a pharmaceutical composition comprising a solid dispersion (e.g., a spray dried solid dispersion), as described herein, and one or more pharmaceutically acceptable excipients selected from microcrystalline cellulose (e.g., Avicel PH 102 from FMC Biopolymer), mannitol (e.g., Parteck M100 Mannitol from EMD Millipore), silica (e.g., Syloid 244 FP from Grace Davison), magnesium stearate (e.g., magnesium stearate 2257 from Mallinckrodt), and crosscarmellose sodium (e.g., Ac-Di-Sol from FMC Biopolymer).

In some embodiments, the pharmaceutical compositions herein may further include additives or ingredients, such as antioxidants (e.g., ascorbyl palmitate, butylated hydroxylanisole (BHA), butylated hydroxytoluene (BHT), α-tocopherols, propyl gallate, and fumaric acid), antimicrobial agents, enzyme inhibitors, stabilizers (e.g., malonic acid), and/or preserving agents.

In one embodiment, the present disclosure provides for a composition comprising a solid dispersion (e.g., a spray dried solid dispersion) comprising amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and HPMC-AS (e.g., in a ratio of 70:30 wt%), as described herein, and 20 to 30 wt% microcrystalline cellulose (e.g., Avicel PH 102 from FMC Biopolymer), 5 to 20 wt% mannitol (e.g., Parteck M100 Mannitol from EMD Millipore), 0 to 5 wt% silica (e.g., Syloid 244 FP from Grace Davison), 0 to 3 wt% magnesium stearate (e.g., magnesium stearate 2257 from Mallinckrodt), and 1 to 10 wt% crosscarmellose sodium (e.g., Ac-Di-Sol from FMC Biopolymer). In another embodiment, the present disclosure provides for a composition comprising a solid dispersion (e.g., a spray dried solid dispersion) comprising amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and HPMC-AS (e.g., in a ratio of 70:30 wt%), as described herein, and 24 to 27 wt% microcrystalline cellulose (e.g., 26 wt% Avicel PH 102 from FMC Biopolymer), 12 to 13 wt% mannitol (e.g., 12.3 wt% Parteck M100 Mannitol from EMD Millipore), 0.5 to 2 wt% silica (e.g., 1 wt% Syloid 244 FP from Grace Davison), 0.3 to 1.0 wt% magnesium stearate (e.g., 0.6 wt% magnesium stearate 2257 from Mallinckrodt), and 4 to 6 wt% crosscarmellose sodium (e.g., 5 wt % Ac-Di-Sol from FMC Biopolymer)

In one embodiment, the pharmaceutical compositions described herein comprise at least 30% by weight, at least 40% by weight, at least 45% by weight, at least 50% by weight, at least 60% by weight, or at least 70% by weight of the solid dispersion. In another embodiment, the pharmaceutical compositions described herein comprise 40 wt% to 60 wt% by weight of the solid dispersion. In yet another embodiment, the pharmaceutical compositions described herein comprise 50 wt% to 51 wt% by weight of the solid dispersion.

Provided compositions may be formulated such that a dosage of between 0.001 - 100 mg/kg body weight/day of the inhibitor can be administered to a patient receiving these compositions. It should also be understood that a specific dosage and treatment regimen for any particular patient will depend upon a variety of factors, including age, body weight, general health, sex, diet, time of administration, rate of excretion, drug combination, the judgment of the treating physician, and the severity of the particular disease being treated. The amount of a provided dispersion in the composition will also depend upon the particular compound in the composition.

In one embodiment, the dosage amount of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide ranges from 10 mg to 1 g such as, e.g., from 25 mg to 750 mg, from 50 mg to 500 mg, from 100 to 300 mg, from 150 mg to 250 mg. In embodiment, the dosage amount of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide is formulated at 200 mg.

Diseases and conditions treatable according to the methods described herein include, but are not limited to, diseases and/or disorders associated with cellular proliferation. In some embodiments, the crystalline forms and compositions thereof described herein are useful in treating diseases and/or disorders associated with misregulation of cell cycle or DNA repair. In some embodiments, the crystalline forms and compositions thereof described herein are useful in treating cancer. Exemplary types of cancer include e.g., adrenal cancer, acinic cell carcinoma, acoustic neuroma, acral lentiginous melanoma, acrospiroma, acute eosinophilic leukemia, acute erythroid leukemia, acute lymphoblastic leukemia, acute megakaryoblastic leukemia, acute monocytic leukemia, acute promyelocytic leukemia, adenocarcinoma, adenoid cystic carcinoma, adenoma, adenomatoid odontogenic tumor, adenosquamous carcinoma, adipose tissue neoplasm, adrenocortical carcinoma, adult T-cell leukemia/lymphoma, aggressive NK-cell leukemia, AIDS-related lymphoma, alveolar rhabdomyosarcoma, alveolar soft part sarcoma, ameloblastic fibroma, anaplastic large cell lymphoma, anaplastic thyroid cancer, angioimmunoblastic T-cell lymphoma, angiomyolipoma, angiosarcoma, astrocytoma, atypical teratoid rhabdoid tumor, B-cell chronic lymphocytic leukemia, B-cell prolymphocytic leukemia, B-cell lymphoma, basal cell carcinoma, biliary tract cancer, bladder cancer, blastoma, bone cancer, Brenner tumor, Brown tumor, Burkitt's lymphoma, breast cancer, brain cancer, carcinoma, carcinoma in situ, carcinosarcoma, cartilage tumor, cementoma, myeloid sarcoma, chondroma, chordoma, choriocarcinoma, choroid plexus papilloma, clear-cell sarcoma of the kidney, craniopharyngioma, cutaneous T-cell lymphoma, cervical cancer, colorectal cancer, Degos disease, desmoplastic small round cell tumor, diffuse large B-cell lymphoma, dysembryoplastic neuroepithelial tumor, dysgerminoma, embryonal carcinoma, endocrine gland neoplasm, endodermal sinus tumor, enteropathy-associated T-cell lymphoma, esophageal cancer, fetus in fetu, fibroma, fibrosarcoma, follicular lymphoma, follicular thyroid cancer, ganglioneuroma, gastrointestinal cancer, germ cell tumor, gestational choriocarcinoma, giant cell fibroblastoma, giant cell tumor of the bone, glial tumor, glioblastoma multiforme, glioma, gliomatosis cerebri, glucagonoma, gonadoblastoma, granulosa cell tumor, gynandroblastoma, gallbladder cancer, gastric cancer, hairy cell leukemia, hemangioblastoma, head and neck cancer, hemangiopericytoma, hematological malignancy, hepatoblastoma, hepatosplenic T-cell lymphoma, Hodgkin's lymphoma, non-Hodgkin's lymphoma, invasive lobular carcinoma, intestinal cancer, kidney cancer, laryngeal cancer, lentigo maligna, lethal midline carcinoma, leukemia, leydig cell tumor, liposarcoma, lung cancer, lymphangioma, lymphangiosarcoma, lymphoepithelioma, lymphoma, acute lymphocytic leukemia, acute myelogenous leukemia, chronic lymphocytic leukemia, liver cancer, small cell lung cancer, non-small cell lung cancer, MALT lymphoma, malignant fibrous histiocytoma, malignant peripheral nerve sheath tumor, malignant triton tumor, mantle cell lymphoma, marginal zone B-cell lymphoma, mast cell leukemia, mediastinal germ cell tumor, medullary carcinoma of the breast, medullary thyroid cancer, medulloblastoma, melanoma, meningioma, merkel cell cancer, mesothelioma, metastatic urothelial carcinoma, mixed Mullerian tumor, mucinous tumor, multiple myeloma, muscle tissue neoplasm, mycosis fungoides, myxoid liposarcoma, myxoma, myxosarcoma, nasopharyngeal carcinoma, neurinoma, neuroblastoma, neurofibroma, neuroma, nodular melanoma, ocular cancer, oligoastrocytoma, oligodendroglioma, oncocytoma, optic nerve sheath meningioma, optic nerve tumor, oral cancer, osteosarcoma, ovarian cancer, Pancoast tumor, papillary thyroid cancer, paraganglioma, pinealoblastoma, pineocytoma, pituicytoma, pituitary adenoma, pituitary tumor, plasmacytoma, polyembryoma, precursor T-lymphoblastic lymphoma, primary central nervous system lymphoma, primary effusion lymphoma, primary peritoneal cancer, prostate cancer, pancreatic cancer, pharyngeal cancer, pseudomyxoma peritonei, renal cell carcinoma, renal medullary carcinoma, retinoblastoma, rhabdomyoma, rhabdomyosarcoma, Richter's transformation, rectal cancer, sarcoma, Schwannomatosis, seminoma, Sertoli cell tumor, sex cord-gonadal stromal tumor, signet ring cell carcinoma, skin cancer, small blue round cell tumors, small cell carcinoma, soft tissue sarcoma, somatostatinoma, soot wart, spinal tumor, splenic marginal zone lymphoma, squamous cell carcinoma, synovial sarcoma, Sezary's disease, small intestine cancer, squamous carcinoma, stomach cancer, T-cell lymphoma, testicular cancer, thecoma, thyroid cancer, transitional cell carcinoma, throat cancer, urachal cancer, urogenital cancer, urothelial carcinoma, uveal melanoma, uterine cancer, verrucous carcinoma, visual pathway glioma, vulvar cancer, vaginal cancer, Waldenstrom's macroglobulinemia, Warthin's tumor, and Wilms' tumor.

In one embodiment, the cancer treated by the solid dispersions and compositions thereof described herein is selected from breast cancer, prostate cancer, colon cancer, renal cell carcinoma, glioblastoma multiforme cancer, bladder cancer, melanoma, bronchial cancer, lymphoma, liver cancer, multiple myeloma, lymphoma, ovarian cancer, NSCL, pancreatic cancers, malignant rhabdoid tumor, synovial sarcoma, and glioma.

Another embodiment of the present disclosure is the use of the solid dispersions as described herein in the manufacture of a medicament for use in the treatment of a disorder or disease herein. Another object of the present disclosure is the solid dispersion or composition described herein for use in the treatment of a disorder or disease herein.

### Processes of Manufacture

The solid dispersions described herein can be prepared by a number of methods, including by melting and solvent evaporation. The solid dispersions of the present invention can also be prepared according to the procedures described in: Chiou WL, Riegelman S: "Pharmaceutical applications of solid dispersion systems", J. Pharm. Sci. 1971; 60: 1281-1302; Serajuddin ATM: "Solid dispersion of poorly water-soluble drugs: early promises, subsequent problems, and recent breakthroughs", J. Pharm. Sci. 1999; 88: 1058-1066; Leuner C, Dressman J: "Improving drug solubility for oral delivery using solid dispersions", Eur. J. Pharm. Biopharm. 2000; 50:47-60; and Vasconcelos T, Sarmento B, Costa P: "Solid dispersions as strategy to improve oral bioavailability of poor water soluble drugs", Drug Discovery Today 2007; 12:1068-1075, all of which are incorporated herein by reference in their entireties. In one embodiment, spray drying preparation is used which involves atomization of a solution of the composition into small droplets, followed by rapid removal solvent from the formulation.

(R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide can be made according to the procedure set forth in U.S. Patent No. 9,085,583.

In one embodiment, a method for preparing the solid dispersions described herein comprise spray drying a solution comprising amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof, and a pharmaceutically acceptable polymer. Suitable solvents for the solution include, but are not limited to, ethanol (EtOH), water, acetone, and isopropanol (IPA). Mixed solvent systems are also included, such as e.g., acetone:water, acetone:EtOH, EtOH:water, IPA:water, acetone:IPA:water, acetone:EtOH:water, etc. In one embodiment, the solvent or solvent system used in the methods for preparing the solid dispersions is acetone or a mixture of acetone and water (acetone:water).

The percentage of each respective solvent in a mixed solvent system can range anywhere from 1 to 99%. Thus, exemplary solvent system ratios include e.g., from 90% to 99% solvent A and from 10% to 1% solvent B, from 80% to 89% solvent A and from 20% to 11% solvent B, from 70% to 79% solvent A and from 30% to 21% solvent B, from 60% to 69% solvent A and from 40% to 31% solvent B, from 50% to 59% solvent A and from 59% to 50% solvent B, and vice versa. For mixtures of three solvents or more, all ratios are contemplated so long as the total percentage adds up to 100%. For example, in the case of a mixed solvent system containing three solvents, an acceptable ratio would include from 40% to 49% solvent A, from 49% to 40% solvent B, and from 2% to 20% solvent C.

In one embodiment, the solvent system of the solution comprises a mixture of 90% to 99% acetone and 10% to 1% water. In another embodiment, the solvent system of the solution comprises a mixture of 94% to 96% acetone and 6% to 4% water. In yet another embodiment, the solvent system of the solution comprises a mixture of 95% acetone and 5% water.

In one embodiment, the total weight percentage of solids in the solid dispersion comprising amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide is greater than or equal to 10 wt% at room temperature (RT). For example, from 10 wt% to 30 wt% solids at RT, from 10 wt% to 20 wt% solids at RT, from 12 wt% to 17 wt% solids at RT, from 13 wt% to 16 wt% solids at RT, or from 14 wt% to 16 wt% solids at RT, or at 15 wt% solids at RT.

In one embodiment, the pharmaceutically acceptable polymer, ratio of amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide to polymer, solvent system and ratio if applicable, and % total of solids in the solution includes those set forth in Table 1.

### EXEMPLIFICATION

### Example 1: Preparation of Spray Dried Dispersions Comprising Amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide

Spray dried dispersions of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide were prepared according to the process outlined in **Figure 9****.** Six different polymers systems were used after initial screening procedures: PVP (PVP K29/32 polymer system), PVP-VA (PVP-VA 64 polymer system), PVP-VA sprayed with sodium bicarbonate (PVP-VA 64 + NaHCO₃ polymer system, 1% NaHCO₃), HPMC (HPMC E3 premium LV polymer system), HPMC-P (e.g., HPMC-P 55 polymer system), HPMC-AS (HPMC-AS-M granular polymer system), and Eudragit.

Except for the dispersion with Eudragit, which was performed at 50 wt% drug load, all spray dried solutions were prepared at 25 wt%, 50 wt%, and 70 wt % drug load, i.e., (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide. Other details, such as solvents systems, % Sfc moisture, DVS % weight gain, achiral purity, etc., for the non-Eudragit systems are provided in **Figures 11-16****.** Details for the initial solvent screen are reproduced below in Table 1. Solution stability testing for Run 21 was found to support up to 24 hours of heating at 50 °C with no changes to chromatographic purity. Although all of the solid dispersions generated herein would be viable candidates for commercial development, HPMC-AS dispersion at 70% API was selected as the lead because of enhanced physical properties, low hygroscopicity, and acceptable pharmacokinetic and stability results. For example, other solid dispersion polymers require higher amounts of super disintegrants and include the use of porosogens to achieve rapid disintegration and dissolutions. The use of porosogens add extra complexity by increasing hygroscopicity and the potential for increased degradation due to increased water activity. Porosogens also increase the manufacturing complexity by making it necessary to control the incoming properties of additional excipient properties. Higher levels of superdisintegrants can also result in increased hygroscopicity, problems associated with pan coating, tablet softening on stability and decreased release rates over time. HPMC and HPMC-AS avoid this.

Dispersions with Eudragit produced irregular morphology, increased solvent content after secondary drying, and had reduced dissolution profiles to HMPC-AS and the PVP and PVP-VA polymer dispersions. The 70% drug load (as compared to lower amounts) was selected to enable higher dosage strength with smaller tablet size.

**Table 1**

| Run # | Solid Dispersion | Solvent System (%) | % Total Solids |
|---|---|---|---|
| 1 | 1:1 API:PVP-VA | EtOH | 20 |
| 2 | 1:1 API:PVP-VA + NaHCO₃ | EtOH:water (80:20) | 20 |
| 3 | 1:1 API:PVP | acetone:EtOH (50:50) | 20 |
| 4 | 1:1 API:Eudragit | acetone:EtOH (50:50) | 20 |
| 5 | 1:1 API:HPMC | acetone:IPA:water (40:40:20) | 20 |
| 6 | 1:1 API:HPMC-P | acetone | 20 |
| 7 | 1:1 API:HPMC-AS | acetone:water (90:10) | 20 |
| 8 | 1:3 API:PVP-VA | EtOH | 20 |
| 9 | 1:3 API:PVP-VA + NaHCO₃ | EtOH:water (80:20) | 20 |
| 10 | 1:3 API:PVP | acetone:EtOH (50:50) | 20 |
| 11 | 1:3 API:HPMC | acetone:IPA:water (40:40:20) | 11.3 |
| 12 | 1:3 API:HPMC-P | acetone | 20 |
| 13 | 1:3 API:HPMC-AS | acetone:water (90:10) | 20 |
| 14 | 7:3 API:PVP-VA | EtOH | 13.3 |
| 15 | 7:3 API:PVP-VA + NaHCO₃ | EtOH:water (95:5) | 13.4 |
| 16 | 7:3 API:PVP | acetone:EtOH (50:50) | 20 |
| 17 | 7:3 API:HPMC | acetone:IPA:water (46:46: 8) | 16.5 |
| 18 | 7:3 API:HPMC-P | acetone | 13.3 |
| 19 | 7:3 API:HPMC-AS | acetone:water (94:6) | 13.7 |
| 20 | 7:3 API:HPMC-AS | acetone:water (95:5) | 15 |
| 21 | 7:3 API:HPMC-AS (300g scale up) | acetone:water (95:5) | 15 |

| | | | |
|---|---|---|---|
| * API = amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide | | | |

A clinical Good Manufacturing Process (GMP) using the process outlined in **Figure** 9 was performed to produce a solid dispersion comprising 70 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and 30 wt% HPMC-AS-M. The formula for the preparation is set forth in Table 2. The processing conditions are set forth in Table 3 and the analytical results are set forth in Table 4.

**Table 2**

| | Unity Composition | |
|---|---|---|
| Component / Grade | Solids mg/g | % w/w |
| API | 700 | 10.50 |
| HPMC-AS-M | 300 | 4.50 |
| acetone | NA | 80.75 |
| water | NA | 4.25 |

| | | |
|---|---|---|
| * API = amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide | | |

**Table 3**

| Buchi Configuration | Negative Pressure | |
|---|---|---|
| Nozzle Configuration | 1.5 mm Air Carp / 0.7 mm Liquid Insert | |
| Formulation | 70 wt% API : 30 wt% HPMC-AS-M | |

| Lot | Lot 1 | Lot 2 |
|---|---|---|
| Batch size (grams) | 500.0 | 557.1 |
| Solution Flow Rate (g/min) | 23 | 23 |
| Atomization Pressure (psi) | 26 | 26 |
| Inlet Temperature (°C) | 68 | 68 |
| Outlet Temperature (°C) | 40 | 40 |
| Aspirator Set Point (%) | 100 | |
| Wet Dispersion Yield (%) | 100 | 101 |
| Dry Dispersion Yield (%) | 94 | 97 |

| | | |
|---|---|---|
| * API = amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide | | |

**Table 4**

| | |
|---|---|
| Appearance | free flowing white powder |
| Residual Acetone | < 200 ppm |
| Potency (HPLC) | 68.5 wt % |
| Amine Impurity (HPLC) | 0.01 wt% |
| XRPD | consistent with amorphous form |
| Modulated Differential Scanning Calorimetry (mDSC) | Average Tg = 115.10 °C |
| Water Content (KF) | 0.82 wt% |
| Particle Size | Dᵥ₁₀ = 3.39 µm |
| | Dᵥ₅₀ = 11.8 µm |
| | Dᵥ₉₀ = 29.7 µm |

### Example 2: Tablet Formation of Spray Dried Dispersion of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide

A 200 mg tablet dosage form comprising a solid dispersion comprising 70 wt% (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and 30 wt% HPMC-AS-M was manufactured according to the procedures set forth in Figure **10****.** The unit composition of the formula is set forth in Table 5.

**Table 5**

| *Intragranular* | | | |
|---|---|---|---|
| | Unit Composition | | |
| **Component** | **mg/tablet** | **% w/w** | **Manufacturer** |
| Solid Dispersion 70:30 API:HPMC-AS-M | 285.72 | 50.12 | NA |
| Avicel PH 102 | 148.20 | 26.00 | FMC Biopolymer |
| Parteck M100 Mannitol | 70.00 | 12.28 | EMD Millipore |
| Syloid 244 FP | 2.85 | 0.50 | Grace Davison |
| Magnesium Stearate | 1.70 | 0.30 | Mallinckrodt |
| * API = amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide | | | |

| *Extragranular* | | | |
|---|---|---|---|
| | Unit Composition | | |
| **Component** | **mg/tablet** | **% w/w** | **Manufacturer** |
| Parteck M100 | 28.50 | 5.00 | EMD Millipore |
| Ac-Di-Sol | 28.50 | 5.00 | FMC Biopolymer |
| Syloid 244 FP | 2.85 | 0.50 | Grace Davison |
| Magnesium Stearate | 1.70 | 0.30 | Mallinckrodt |

### Example 3: In Vivo Screening of Solid Dispersions of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide

Several rounds of pharmacokinetic studies (PK) were performed and included four diverse solid dispersions comprising 50 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and PVP, PVP-PA with NaHCO₃, HPMC, and HPMC-AS polymer systems, in fasted, famotidine treated dogs at a 30 mg/kg dose. See **Figure 17****.** HPMC-AS and HPMC at 70 wt% drug load were tested in a second round of PK studies and were concluded to be essentially identical in terms of PK. See Table 6.

**Table 6**

| **Dose/Conditions** | **Fed/Fasted** | **Form of API** | **AUC (0 to last) ng/ml - hr** | **AUC/actual dose** | **F%** |
|---|---|---|---|---|---|
| Pentagastrin pretreat, 30 mpk, 3X100 mg DIC/dog, fasted | Fasted | API, 3 X 100 mg in hard gelatin capsule (size 0) | 16392 | 571 | 72 |
| Pentagastrin pretreat, 30 mpk, fasted | Fasted | crystalline form of API, 0.5 MC/0.1 tween suspension | 14425 | 492 | 62 |
| Famotidine pretreat, 30 mpk, fasted | Fasted | crystalline form of API, 0.5 MC/0.1 tween suspension | 448 | 15 | 2 |
| Famotidine pretreat, 30 mpk, fasted | Fasted | Vitamin E TPGS solution | 184483 | 641 | 81 |
| Famotidine pretreat, 30 mpk, fasted | Fasted | Solid dispersion - 50:50 API:PVP-VA w/NaHCO₃ | 4862 | 188 | 24 |
| Famotidine pretreat, 30 mpk, fasted | Fasted | Solid dispersion - 50:50 API:PVP | 11868 | 469 | 59 |
| Famotidine pretreat, 30 mpk, fasted | Fasted | Solid dispersion - 50:50 API:HPMC-AS | 8128 | 268 | 34 |
| Famotidine pretreat, 30 mpk, fasted | Fasted | Solid dispersion - 50:50 API:HPMC | 10157 | 390 | 49 |
| Famotidine pretreat, 30 mpk, fasted | Fasted | Solid dispersion - 50:50 API:HPMC-AS | 12360 | 412 | 52 |
| Famotidine pretreat, 30 mpk, fasted | Fasted | Solid dispersion - 70:30 API:HPMC | 8970 | 299 | 38 |
| Pentagastrin, 30 mpk, fasted | Fasted | Solid dispersion - 70:30 API:HPMC-AS | 17658 | 600 | 75 |

| | | | | | |
|---|---|---|---|---|---|
| * Unless indicated otherwise, API = amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide | | | | | |

As shown by **Figures 17** and **18****,** all of the solid dispersions demonstrated better exposure than the crystalline suspension of (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide in famotidine treated dogs. **Figure 1** shows that 70 wt% amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide and 30 wt% HPMC-AS profile remained unchanged when give to dogs pre-treated with either famotidine or pentagastrin, while the crystalline form (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide toxicology formulation shows a clear drop in exposure with famotidine pretreatment.

All solid dispersions were prepared as aqueous suspensions at 60 mg/mL in the toxicology vehicle, 0.5% MC/0.1% Tween 80 and does with 30 minutes of preparation. All dogs tested were carried over from study to study.

Unless otherwise defined, all technical and scientific terms used herein are accorded the meaning commonly known to one with ordinary skill in the art.

## Claims

1. A solid dispersion comprising amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or a pharmaceutically acceptable salt thereof; and a pharmaceutically acceptable polymer, wherein the pharmaceutically acceptable polymer is selected from polyvinylpyrrolidone (PVP), polyvinylpyrrolidone/vinyl acetate copolymer (PVP-VA), hydroxypropyl methylcellulose (HPMC), hypromellose phthalate (HPMC-P), and hypromellose acetate succinate (HPMC-AS).

2. The solid dispersion of Claim 1, wherein the pharmaceutically acceptable polymer is HPMC or HPMC-AS, preferably HPMC-AS grade M.

3. The solid dispersion of any one of Claims 1 to 2, wherein the weight ratio of the pharmaceutically acceptable polymer to (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide ranges from 10:90 wt% to 90:10 wt%, from 15:85 wt% to 85:15 wt%, from 20:80 wt% to 80:20 wt%, from 25:75 wt% to 75:25 wt%, from 30:70 wt% to 70:30 wt%, from 35:65 wt% to 65:35 wt%, from 40:60 wt% to 60:40 wt%, or from 45:55 wt% to 55:45 wt%.

4. The solid dispersion of any one of Claims 1 to 3, wherein the weight ratio of the pharmaceutically acceptable polymer to (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide is 30:70 wt%.

5. The solid dispersion of claim 4, wherein the pharmaceutically acceptable polymer is HPMC-AS.

6. The solid dispersion of any one of Claims 1 to 5, wherein the solid dispersion comprises a particle size (Dv₅₀) ranging from 5 µM to 20 µM, preferably from 10 µM to 12 µM.

7. A pharmaceutical composition comprising the solid dispersion of any one of Claims 1 to 6; and one or more pharmaceutically acceptable excipients.

8. The pharmaceutical composition of Claim 7, wherein the excipients are selected from microcrystalline cellulose, mannitol, silica, magnesium stearate, and crosscarmellose sodium.

9. The pharmaceutical composition of Claim 7 or 8, comprising 20 to 30 wt% microcrystalline cellulose, 5 to 20 wt% mannitol, 0 to 5 wt% silica, 0 to 3 wt% magnesium stearate, and 1 to 10 wt% crosscarmellose sodium.

10. The pharmaceutical composition of any one of Claims 7 to 9, comprising 40 wt% to 60 wt% by weight of the solid dispersion, preferably 50 wt% to 51 wt% by weight of the solid dispersion.

11. The solid dispersion of any one of Claims 1 to 6, or the pharmaceutical compositions of any one of Claims 7 to 10 for use in treating a disease or disorder associated with cellular proliferation.

12. The solid dispersion of any one of claims 1 to 6, or the pharmaceutical compositions of any one of claims 7 to 10 for use of Claim 11, wherein the disease is cancer.

13. The solid dispersion of any one of claims 1 to 6, or the pharmaceutical compositions of any one of claims 7 to 10 for use of Claim 12, wherein the cancer is selected from breast cancer, prostate cancer, colon cancer, renal cell carcinoma, glioblastoma multiforme cancer, bladder cancer, melanoma, bronchial cancer, lymphoma, and liver cancer.

14. A method of preparing a solid dispersion of any one of Claims 1 to 6, comprising spray drying a solution comprising amorphous (R)-N-((4-methoxy-6-methyl-2-oxo-1,2-dihydropyridin-3-yl)methyl)-2-methyl-1-(1-(1-(2,2,2-trifluoroethyl)piperidin-4-yl)ethyl)-1H-indole-3-carboxamide, or the pharmaceutically acceptable salt thereof, and the pharmaceutically acceptable polymer.

15. The method of Claim 14, wherein the solution comprises a mixture of acetone and water.

## Patentansprüche

1. Feste Dispersion, umfassend amorphes (R)-N-((4-Methoxy-6-methyl-2-oxo-1,2-dihydropyridin-3-yl)methyl)-2-methyl-1-(1-(1-(2,2,2-trifluorethyl)piperidin-4-yl)ethyl)-1H-indol-3-carboxamid, oder ein pharmazeutisch akzeptables Salz davon; und ein pharmazeutisch akzeptables Polymer, wobei das pharmazeutisch akzeptable Polymer ausgewählt ist aus Polyvinylpyrrolidon (PVP), Polyvinylpyrrolidon/Vinylacetat-Copolymer (PVP-VA), Hydroxypropylmethylcellulose (HPMC), Hypromellosephthalat (HPMC-P) und Hypromelloseacetatsuccinat (HPMC-AS).

2. Feste Dispersion nach Anspruch 1, wobei das pharmazeutisch akzeptable Polymer HPMC oder HPMC-AS, vorzugsweise HPMC-AS Grad M, ist.

3. Feste Dispersion nach einem der Ansprüche 1 bis 2, wobei das Gewichtsverhältnis des pharmazeutisch akzeptablen Polymers zu (R)-N-((4-Methoxy-6-methyl-2-oxo-1,2-dihydropyridin-3-yl)methyl)-2-methyl-1-(1-(1-(2,2,2-trifluorethyl)piperidin-4-yl)ethyl)-1H-indol-3-carboxamid im Bereich von 10:90 Gew.-% bis 90:10 Gew.-%, von 15:85 Gew.-% bis 85:15 Gew.-%, von 20:80 Gew.-% bis 80:20 Gew.-%, von 25:75 Gew.-% bis 75:25 Gew.-%, von 30:70 Gew.-% bis 70:30 Gew.-%, von 35:65 Gew.-% bis 65:35 Gew.-%, von 40:60 Gew.-% bis 60:40 Gew.-% oder von 45:55 Gew.-% bis 55:45 Gew.-% liegt.

4. Feste Dispersion nach einem der Ansprüche 1 bis 3, wobei das Gewichtsverhältnis des pharmazeutisch akzeptablen Polymers zu (R)-N-((4-Methoxy-6-methyl-2-oxo-1,2-dihydropyridin-3-yl)methyl)-2-methyl-1-(1-(1-(2,2,2-trifluorethyl)piperidin-4-yl)ethyl)-1H-indol-3-carboxamid 30:70 Gew.-% beträgt.

5. Feste Dispersion nach Anspruch 4, wobei das pharmazeutisch akzeptable Polymer HPMC-AS ist.

6. Feste Dispersion nach einem der Ansprüche 1 bis 5, wobei die feste Dispersion eine Teilchengröße (Dv₅₀) im Bereich von 5 µM bis 20 µM, vorzugsweise von 10 µM bis 12 µM, aufweist.

7. Pharmazeutische Zusammensetzung, umfassend die feste Dispersion nach einem der Ansprüche 1 bis 6; und einen oder mehrere pharmazeutisch akzeptable Hilfsstoffe.

8. Pharmazeutische Zusammensetzung nach Anspruch 7, wobei die Hilfsstoffe ausgewählt sind aus mikrokristalliner Cellulose, Mannitol, Siliciumdioxid, Magnesiumstearat und Croscarmellose-Natrium.

9. Pharmazeutische Zusammensetzung nach Anspruch 7 oder 8, umfassend 20 bis 30 Gew.-% mikrokristalline Cellulose, 5 bis 20 Gew.-% Mannitol, 0 bis 5 Gew.-% Siliciumdioxid, 0 bis 3 Gew.-% Magnesiumstearat und 1 bis 10 Gew.-% Croscarmellose-Natrium.

10. Pharmazeutische Zusammensetzung nach einem der Ansprüche 7 bis 9, umfassend 40 Gew.-% bis 60 Gew.-% der festen Dispersion, vorzugsweise 50 Gew.-% bis 51 Gew.-% der festen Dispersion.

11. Die feste Dispersion nach einem der Ansprüche 1 bis 6 oder die pharmazeutischen Zusammensetzungen nach einem der Ansprüche 7 bis 10 zur Verwendung beim Behandeln einer mit der Zellproliferation assoziierten Krankheit oder Störung.

12. Die feste Dispersion nach einem der Ansprüche 1 bis 6 oder die pharmazeutischen Zusammensetzungen nach einem der Ansprüche 7 bis 10 zur Verwendung nach Anspruch 11, wobei die Krankheit Krebs ist.

13. Die feste Dispersion nach einem der Ansprüche 1 bis 6 oder die pharmazeutischen Zusammensetzungen nach einem der Ansprüche 7 bis 10 zur Verwendung nach Anspruch 12, wobei der Krebs ausgewählt ist aus Brustkrebs, Prostatakrebs, Dickdarmkrebs, Nierenzellkarzinom, Glioblastoma multiforme, Blasenkrebs, Melanom, Bronchialkrebs, Lymphom und Leberkrebs.

14. Verfahren zur Herstellung einer festen Dispersion nach einem der Ansprüche 1 bis 6, umfassend das Sprühtrocknen einer Lösung, die amorphes (R)-N-((4-Methoxy-6-methyl-2-oxo-1,2-dihydropyridin-3-yl)methyl)-2-methyl-1-(1-(1-(2,2,2-trifluorethyl)piperidin-4-yl)ethyl)-1H-indol-3-carboxamid oder das pharmazeutisch akzeptable Salz davon und das pharmazeutisch akzeptable Polymer umfasst.

15. Verfahren nach Anspruch 14, wobei die Lösung ein Gemisch aus Aceton und Wasser umfasst.

## Revendications

1. Dispersion solide comprenant du (R)-N-((4-méthoxy-6-méthyl-2-oxo-1,2-dihydropyridin-3-yl)méthyl)-2-méthyl-1-(1-(1-(2,2,2-trifluoroéthyl)pipéridin-4-yl)éthyl)-1H-indole-3-carboxamide amorphe ou un sel pharmaceutiquement acceptable de celui-ci ; et un polymère pharmaceutiquement acceptable, le polymère pharmaceutiquement acceptable étant choisi parmi la polyvinylpyrrolidone (PVP), un copolymère polyvinylpyrrolidone/acétate de vinyle (PVP-VA), l'hydroxypropylméthylcellulose (HPMC), le phtalate d'hypromellose (HPMC-P) et l'acétate et succinate d'hypromellose (HPMC-AS).

2. Dispersion solide selon la revendication 1, dans laquelle le polymère pharmaceutiquement acceptable est le HPMC ou le HPMC-AS, de préférence le HPMC-AS grade M.

3. Dispersion solide selon l'une des revendications 1 à 2, dans laquelle le rapport en poids du polymère pharmaceutiquement acceptable au (R)-N-((4-méthoxy-6-méthyl-2-oxo-1,2-dihydropyridin-3-yl)méthyl)-2-méthyl-1-(1-(1-(2,2,2-trifluoroéthyl)pipéridin-4-yl)éthyl)-1H-indole-3-carboxamide est compris dans la plage de 10:90 % en poids à 90:10 % en poids, de 15:85 % en poids à 85:15 % en poids, de 20:80 % en poids à 80:20 % en poids, de 25:75 % en poids à 75:25 % en poids, de 30:70 % en poids à 70:30 % en poids, de 35:65 % en poids à 65:35 % en poids, de 40:60 % en poids à 60:40 % en poids ou de 45:55 % en poids à 55:45 % en poids.

4. Dispersion solide selon l'une des revendications 1 à 3, dans laquelle le rapport en poids du polymère pharmaceutiquement acceptable au (R)-N-((4-méthoxy-6-méthyl-2-oxo-1,2-dihydropyridin-3-yl)méthyl)-2-méthyl-1-(1-(1-(2,2,2-trifluoroéthyl)pipéridin-4-yl)éthyl)-1H-indole-3-carboxamide est de 30:70 % en poids.

5. Dispersion solide selon la revendication 4, dans laquelle le polymère pharmaceutiquement acceptable est le HPMC-AS.

6. Dispersion solide selon l'une des revendications 1 à 5, dans laquelle la dispersion solide présente une granulométrie (Dv₅₀) comprise dans la plage de 5 µm à 20 µm, de préférence de 10 µm à 12 µm.

7. Composition pharmaceutique comprenant la dispersion solide selon l'une des revendications 1 à 6 ; et un ou plusieurs excipients pharmaceutiquement acceptables.

8. Composition pharmaceutique selon la revendication 7, dans laquelle les excipients sont choisis parmi la cellulose microcristalline, le mannitol, la silice, le stéarate de magnésium, et la croscarmellose sodique.

9. Composition pharmaceutique selon la revendication 7 ou 8, comprenant 20 à 30 % en poids de cellulose microcristalline, 5 à 20 % en poids de mannitol, 0 à 5 % en poids de silice, 0 à 3 % en poids de stéarate de magnésium et 1 à 10 % en poids de croscarmellose sodique.

10. Composition pharmaceutique selon l'une des revendications 7 à 9, comprenant 40 % en poids à 60 % en poids de la dispersion solide, de préférence 50 % en poids à 51 % en poids de la dispersion solide.

11. Dispersion solide selon l'une des revendications 1 à 6, ou composition pharmaceutique selon l'une des revendications 7 à 10, pour une utilisation dans le traitement d'une maladie ou d'un trouble associé à une prolifération cellulaire.

12. Dispersion solide selon l'une des revendications 1 à 6, ou composition pharmaceutique selon l'une des revendications 7 à 10 pour une utilisation selon la revendication 11, la maladie étant un cancer.

13. Dispersion solide selon l'une des revendications 1 à 6, ou composition pharmaceutique selon l'une des revendications 7 à 10 pour une utilisation selon la revendication 12, le cancer étant sélectionné parmi le cancer du sein, le cancer de la prostate, le cancer du côlon, le carcinome des cellules rénales, le cancer glioblastome multiforme, le cancer de la vessie, le mélanome, le cancer des bronches, le lymphome et le cancer du foie.

14. Procédé de préparation d'une dispersion solide selon l'une des revendications 1 à 6, comprenant la lyophilisation d'une solution comprenant du (R)-N-((4-méthoxy-6-méthyl-2-oxo-1,2-dihydropyridin-3-yl)méthyl)-2-méthyl-1-(1-(1-(2,2,2-trifluoroéthyl)pipéridin-4-yl)éthyl)-1H-indole-3-carboxamide amorphe, ou le sel pharmaceutiquement acceptable de celui-ci, et le polymère pharmaceutiquement acceptable.

15. Procédé selon la revendication 14, dans lequel la solution comprend un mélange d'acétone et d'eau.
